# EUROPEAN PATENT APPLICATION

(11) **EP 2 233 459 A1**
(43) Date of publication of application: **29.09.2010**
(21) Application number: 08863898.6
(22) Date of filing: 17.12.2008
(51) Int. Cl.: C07B 37/04, C07C 1/26, C07C 15/14, C07C 17/269, C07C 25/18, C07C 41/30, C07C 43/205, C07C 45/72, C07C 49/784, C07C 253/30, C07C 255/51, C07C 303/30, C07C 303/40, C07C 309/73, C07C 311/16, C07D 215/06, C07D 333/10, C07B 61/00, C08G 61/12

(54) **METHOD FOR PRODUCING CONJUGATED AROMATIC COMPOUND**

(30) Priority: 21.12.2007 JP 2007329893
(71) Applicant: Sumitomo Chemical Company, Limited, Tokyo 104-8260 (JP)
(72) Inventor: ODA, Seiji, Ibaraki-shi Osaka 567-0841 (JP); KAMIKAWA, Takashi, Nara-shi Nara 630-8442 (JP)
(74) Representative: Vossius & Partner
(86) International application number: PCT/JP2008/073495
(87) International publication number: WO 2009/081956

(57) **Abstract**

A method for producing a conjugated aromatic compound comprising reacting an aromatic compound (A) wherein one or two leaving groups are bonded to an aromatic ring with an aromatic compound (A) having the same structure as that of the above-mentioned aromatic compound (A) or an aromatic compound (B) being structurally different from the above-mentioned aromatic compound (A) and having one or two leaving groups bonded to an aromatic ring, in the presence of a nickel compound, a ligand, a manganese salt and a metal reducing agent.

## Description

### Technical Field

The present invention relates to a method for producing a conjugated aromatic compound.

### Background Art

In Macromolecules 1992, 25, 1214-1223, a homo-coupling reaction of an aromatic dihalide compound in the presence of a zero-valent nickel compound is disclosed. In Tetrahedron Letters 1977, 47, 4089-4092, a coupling reaction of an aromatic dihalide compound in the presence of nickel chloride, triphenylphosphine and zinc.

### Disclosure of the Invention

The present invention provides:
<1> A method for producing a conjugated aromatic compound comprising reacting an aromatic compound (A) wherein one or two leaving groups are bonded to an aromatic ring with an aromatic compound (A) having the same structure as that of the above-mentioned aromatic compound (A) or an aromatic compound (B) being structurally different from the above-mentioned aromatic compound (A) and having one or two leaving groups bonded to an aromatic ring, in the presence of a nickel compound, a ligand, a manganese salt and a metal reducing agent;
<2> The method according to <1>, wherein the aromatic rings of the aromatic compounds (A) and (B) are independently a benzene ring, a biphenyl ring, a naphthalene ring, a fluorene ring, an anthracene ring, a phenanthrene ring, a thiophene ring, a pyrrole ring, a pyridine ring, a pyrimidine ring, a quinoline ring, an isoquinoline ring or a quinoxaline ring, and the aromatic ring may be substituted with at least one group uninvolved in the reaction;
<3> The method according to <1> or <2>, wherein an aromatic compound (A) is reacted with an aromatic compound (A) having the same structure as that of the aromatic compound (A);
<4> The method according to <1> or <2>, wherein the aromatic compound (A) is reacted with an aromatic compound (B) being structurally different from the aromatic compound (A);
<5> The method according to <3> or <4>, wherein the aromatic compound (A) is an aromatic compound represented by the formula (2) :

   Ar^{1_}(X¹)ₙ (2)

   wherein Ar¹ represents an n-valent aromatic group, and the aromatic ring of which the above-mentioned aromatic group is composed is a benzene ring, a biphenyl ring, a naphthalene ring, a fluorene ring, an anthracene ring, a phenanthrene ring, a thiophene ring, a pyrrole ring, a pyridine ring, a pyrimidine ring, a quinoline ring, an isoquinoline ring or a quinoxaline ring, and may be substituted with at least one group uninvolved in the reaction, X¹ is independently in each occurrence a leaving group, and n represents 1 or 2;
<6> The method according to <3> or <4>, wherein the aromatic compound (A) is an aromatic compound represented by the formula (3): 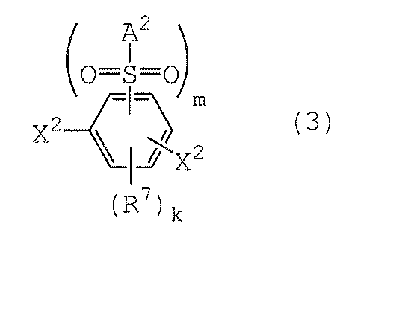 wherein A² represents an amino group substituted with one or two C1-C20 hydrocarbon groups, or a C1-C20 alkoxy group, and the above-mentioned hydrocarbon and alkoxy groups may be substituted with at least one group selected from the group consisting of a fluorine atom, a C1-C20 alkoxy group, a C6-C20 aryl group, a C6-C20 aryloxy group, a C2-C20 acyl group and a cyano group, R⁷ is independently in each occurrence a hydrogen atom, a fluorine atom, a C1-C20 alkyl group, a C1-C20 alkoxy group, a C6-C20 aryl group, a C6-C20 aryloxy group, a C2-C20 acyl group or a cyano group, and the above-mentioned C1-C20 alkyl, C1-C20 alkoxy, C6-C20 aryl, C6-C20 aryloxy and C2-C20 acyl groups may be substituted with at least one substituent selected from the group consisting of a fluorine atom, a cyano group, a C1-C20 alkoxy group, a C6-C20 aryl group and a C6-C20 aryloxy group, and the neighboring two R⁷s may be bonded to form a ring, X² represents a chlorine atom, a bromine atom or an iodine atom, and m represents 1 or 2 and k represents 4-m;
<7> The method according to <3> or <4>, wherein the aromatic compound (A) is an aromatic compound represented by the formula (4) : 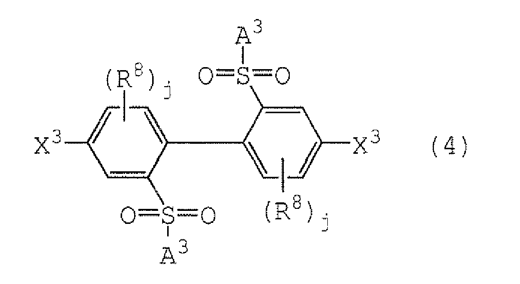 wherein A₃ represents an amino group substituted with one or two C1-C20 hydrocarbon groups, or a C3-C20 alkoxy group, and the above-mentioned hydrocarbon and alkoxy groups may be substituted with at least one group selected from the group consisting of a fluorine atom, a C1-C20 alkoxy group, a C6-C20 aryl group, a C6-C20 aryloxy group, a C2-C20 acyl group and a cyano group, R⁸ is independently in each occurrence a fluorine atom, a C1-C20 alkyl group, a C1-C20 alkoxy group, a C6-C20 aryl group, a C6-C20 aryloxy group, a C2-C20 acyl group or a cyano group, and the above-mentioned C1-C20 alkyl, C1-C20 alkoxy, C6-C20 aryl, C6-C20 aryloxy and C2-C20 acyl groups may be substituted with at least one substituent selected from the group consisting of a fluorine atom, a cyano group, a C1-C20 alkoxy group, a C6-C20 aryl group and a C6-C20 aryloxy group, and the neighboring two R⁸s may be bonded to form a ring,
   X³ represents a chlorine atom, a bromine atom or an iodine atom, and j represents an integer of 0 to 3;
<8> The method according to <4>, wherein an aromatic compound represented by the formula (2):

   Ar^{1_}(X¹)ₙ (2)

   wherein Ar¹, X¹ and n are the same as defined in <5>, is used as the aromatic compound (A), and an aromatic compound represented by the formula (2), an aromatic compound represented by the formula (3): 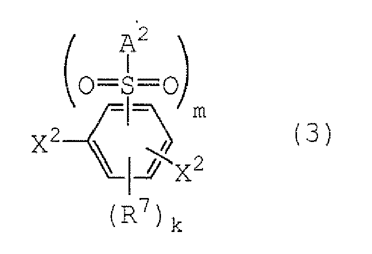 wherein A², R⁷, X², m and k are the same as defined in <6>, an aromatic compound represented by the formula (4): 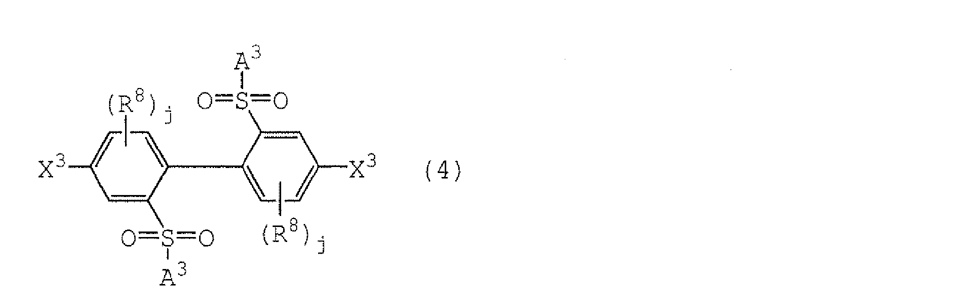 wherein A³, R⁸, X³ and j are the same as defined in <7>, or an aromatic compound represented by the formula (5): 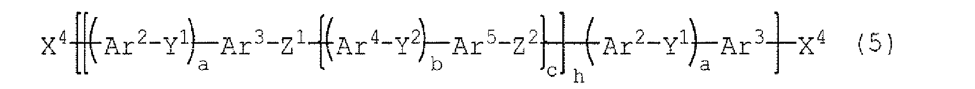 wherein a, b and c are the same or different and represent 0 or 1, and h represents an integer of 5 or more,
   Ar², Ar³, Ar⁴ and Ar⁵ each independently represent a divalent aromatic group, and the divalent aromatic group may be substituted with at least one substituent selected from the group consisting of the following (a2) to (e2):
   (a2) a C1-C20 alkyl group which may be substituted with at least one substituent selected from the group consisting of a fluorine atom, a cyano group, a C1-C20 alkoxy group, a C6-C20 aryl group and a C6-C20 aryloxy group;
   (b2) a C1-C20 alkoxy group which may be substituted with at least one substituent selected from the group consisting of a fluorine atom, a cyano group, a C1-C20 alkoxy group, a C6-C20 aryl group and a C6-C20 aryloxy group;
   (c2) a C6-C20 aryl group which may be substituted with at least one substituent selected from the group consisting of a fluorine atom, a cyano group, a C1-C20 alkoxy group and a C6-C10 aryloxy group;
   (d2) a C6-C20 aryloxy group which may be substituted with at least one substituent selected from the group consisting of a fluorine atom, a cyano group, a C1-C20 alkoxy group and a C6-C20 aryloxy group; and
   (e2) a C2-C20 acyl group which may be substituted with at least one substituent selected from the group consisting of a fluorine atom, a cyano group, a C1-C20 alkoxy group, a C6-C20 aryl group and a C6-C20 aryloxy group, Y¹ and Y² each independently represent a single bond, -CO-, -SO₂-, -C(CH₃)₂-, -C(CF₃)₂- or a fluorene-9,9-diyl group,
      Z¹ and Z² each independently represent -O- or -S-, and X⁴ represents a chlorine atom, a bromine atom or an iodine atom, which are structurally different from the aromatic compound (A), is used as the aromatic compound (B);
<9> The method according to <4>, wherein an aromatic compound represented by the formula (3): 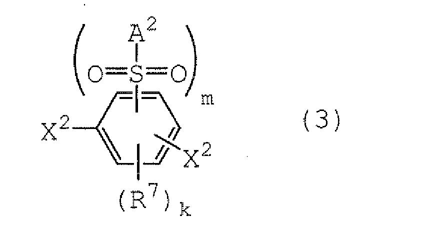 wherein A², R⁷, X², m and k are the same as defined in <6>, is used as the aromatic compound (A), and an aromatic compound represented by the formula (2):

   Ar^{1_}(X¹)ₙ (2)

   wherein Ar¹, X¹ and n are the same as defined in <5>, an aromatic compound represented by the formula (3), an aromatic compound represented by the formula (4): 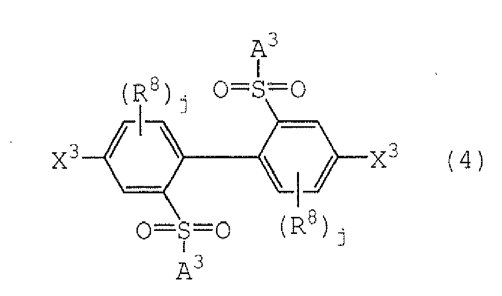 wherein A³, R⁸, X³ and j are the same as defined in <7>, or an aromatic compound represented by the formula (5): 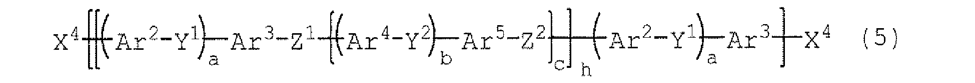 wherein a, b, c, h, Ar², Ar³, Ar⁴, Ar⁵, Y¹, Y², Z¹, Z² and X⁴ are the same as defined in <8>, which are structurally different from the aromatic compound (A), is used as the aromatic compound (B) ;
<10> The method according to <4>, wherein an aromatic compound represented by the formula (4): 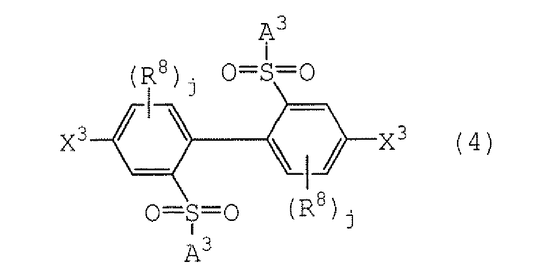 wherein A³, R⁸, X³ and j are the same as defined in <7>, is used as the aromatic compound (A), and an aromatic compound represented by the formula (2):

   Ar^{1_}(X¹)ₙ (2)

   wherein Ar¹, X¹ and n are the same as defined in <5>, an aromatic compound represented by the formula (3): 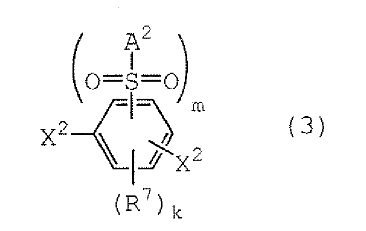 wherein A², R⁷, X², m and k are the same as defined in <6>, an aromatic compound represented by the formula (4) or an aromatic compound represented by the formula (5): 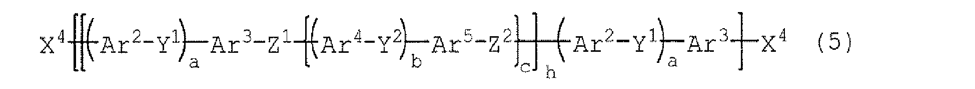 wherein a, b, c, h, Ar², Ar³, Ar⁴, Ar⁵, Y¹, Y², Z¹, Z² and X⁴ are the same as defined in <8>, which are structurally different from the aromatic compound (A), is used as the aromatic compound (B) ;
<11> The method according to any of <1> to <10>, wherein the leaving group is a chlorine atom, a bromine atom or an iodine atom;
<12> The method according to any of <1> to <11>, wherein the nickel compound is a nickel halide;
<13> The method according to any of <1> to <11>, wherein the nickel compound is bis(cyclooctadiene)nickel(0);
<14> The method according to any of <1> to <13>, wherein the ligand is a ligand having a nitrogen atom or a phosphorus atom;
<15> The method according to any of <1> to <14>, wherein the manganese salt is a manganese(II) halide;
<16> The method according to any of <1> to <15>, wherein the metal reducing agent is zinc or manganese.

### Best Mode for Carrying Out the Present Invention

The aromatic compound (A) and the aromatic compound (B) compounds are compounds wherein they have at least one aromatic ring and one or two leaving groups are bonded to an aromatic ring.

The aromatic compound (B) is structurally different from the aromatic compound (A). Hereinafter, the aromatic compounds (A) and (B) are sometimes collectively described as the aromatic compound.

Examples of the aromatic ring include an aromatic hydrocarbon ring such as a benzene ring, a biphenyl ring, a naphthalene ring, a fluorene ring, an anthracene ring and a phenanthrene ring, and a heteroaromatic ring such as a thiophene ring, a pyrrole ring, a pyridine ring, a pyrimidine ring, a quinoline ring, an isoquinoline ring and a quinoxaline ring.

The aromatic ring may be substituted with at least one group uninvolved in the reaction, and specific examples of the group uninvolved in the reaction include the following (a1) to (g1)
(a1) a C1-C20 alkyl group which may be substituted with at least one substituent selected from the group consisting of a fluorine atom, a cyano group, a C1-C20 alkoxy group, a C6-C20 aryl group and a C6-C20 aryloxy group;
(b1) a C1-C20 alkoxy group which may be substituted with at least one substituent selected from the group consisting of a fluorine atom, a cyano group, a C1-C20 alkoxy group, a C6-C20 aryl group and a C6-C20 aryloxy group;
(c1) a C6-C20 aryl group which may be substituted with at least one substituent selected from the group consisting of a fluorine atom, a cyano group, a C1-C20 alkoxy group and a C6-C10 aryloxy group;
(d1) a C6-C20 aryloxy group which may be substituted with at least one substituent selected from the group consisting of a fluorine atom, a cyano group, a C1-C20 alkoxy group and a C6-C20 aryloxy group;
(e1) a C2-C20 acyl group which may be substituted with at least one substituent selected from the group consisting of a fluorine atom, a cyano group, a C1-C20 alkoxy group, a C6-C20 aryl group and a C6-C20 aryloxy group;
(f1) a C2-C20 acyloxy group which may be substituted with at least one substituent selected from the group consisting of a fluorine atom, a cyano group, a C1-C20 alkoxy group, a C6-C20 aryl group and a C6-C20 aryloxy group;
(g1) a C6-C20 arylsulfonyl group which may be substituted with at least one substituent selected from the group consisting of a fluorine atom, a cyano group, a C1-C20 alkoxy group, a C6-C20 aryl group and a C6-C20 aryloxy group; and
(h1) a group represented by the following formula: 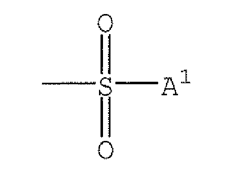 wherein A¹ represents an amino group substituted with one or two C1-C20 hydrocarbon groups, or a C1-C20 alkoxy group, and the above-mentioned hydrocarbon group and the above-mentioned alkoxy group may be substituted with at least one group selected from the group consisting of a fluorine atom, a C1-C20 alkoxy group, a C6-C20 aryl group, a C6-C20 aryloxy group, a C2-C20 acyl group and a cyano group;
   (i1) a cyano group; and
   (j1) a fluorine atom.

Examples of the C1-C20 alkoxy group in (a1) to (h1) include a methoxy group, an ethoxy group, an n-propoxy group, an isopropoxy group, an n-butoxy group, a sec-butoxy group, a tert-butoxy group, an n-pentyloxy group, a 2,2-dimethylpropoxy group, an n-hexyloxy group, a cyclohexyloxy group, an n-heptyloxy group, an n-octyloxy group, an n-nonyloxy group, an n-decyloxy group, an n-undecyloxy group, an n-dodecyloxy group, an n-tridecyloxy group, an n-tetradecyloxy group, an n-pentadecyloxy group, an n-hexadecyloxy group, an n-heptadecyloxy group, an n-octadecyloxy group, an n-nonadecyloxy group and an n-icosyloxy group, and a C1-C6 alkoxy group is preferable.

Examples of the C6-C20 aryl group in (a1) to (h1) include a phenyl group, a 4-methylphenyl group, a 2-methylphenyl group, a 1-naphthyl group, a 2-naphthyl group, a 3-phenanthryl group and a 2-anthryl group.

Examples of the C6-C20 aryloxy group in (a1) to (h1) include those composed of the above-mentioned C6-C20 aryl group and an oxygen atom such as a phenoxy group, a 4-methylphenoxy group, a 2-methylphenoxy group, a 1-naphthyloxy group, a 2-naphthyloxy group, a 3-phenanthryloxy group and a 2-anthryloxy group.

Examples of the C1-C20 alkyl group in (a1) include a methyl group, an ethyl group, an n-propyl group, an isopropyl group, an n-butyl group, an isobutyl group, a sec-butyl group, a tert-butyl group, an n-pentyl group, a 2,2-methylpropyl group, a cyclopentyl group, an n-hexyl group, a cyclohexyl group, an n-heptyl group, a 2-methylpentyl group, an n-octyl group, a 2-ethylhexyl group, an n-nonyl group, an n-decyl group, an n-undecyl group, an n-dodecyl group, an n-tridecyl group, an n-tetradecyl group, an n-pentadecyl group, an n-hexadecyl group, an n-heptadecyl group, an n-octadecyl group, an n-nonadecyl group and an n-icosyl group.

Examples of the C2-C20 acyl group in (e1) and (h1) include a C2-C20 aliphatic or aromatic acyl group such as an acetyl group, a propionyl group, a butyryl group, an isobutyryl group, a benzoyl group, a 1-naphthoyl group and a 2-naphthoyl group.

Examples of the C2-C20 acyloxy group in (f1) include those composed of the above-mentioned C2-C20 acyl group and an oxygen atom such as an acetyloxy group, a propionyloxy group, a butyryloxy group, an isobutyryloxy group, a benzoyloxy group, a 1-naphthoyloxy group and a 2-naphthoyloxy group.

Examples of the C6-C20 arylsulfonyl group in (g1) include a phenylsulfonyl group and a p-toluenesulfonyl group.

Examples of the C1-C20 hydrocarbon group in (h1) include a methyl group, an ethyl group, an n-propyl group, an isopropyl group, an n-butyl group, an isobutyl group, a sec-butyl group, a tert-butyl group, an n-pentyl group, a 2,2-methylpropyl group, an n-hexyl group, a cyclohexyl group, an n-heptyl group, an n-octyl group, an n-nonyl group, an n-decyl group, an n-undecyl group, an n-dodecyl group, an n-tridecyl group, an n-tetradecyl group, an n-pentadecyl group, an n-hexadecyl group, an n-heptadecyl group, an n-octadecyl group, an n-nonadecyl group, an n-icosyl group, a phenyl group, a 1,3-butadiene-1,4-diyl group, a butane-1,4-diyl group, a pentane-1,5-diyl group, a biphenyl-2,2'-diyl group and an o-xylylene group. Examples of the amino group substituted with one or two C1-C20 hydrocarbon groups include a methylamino group, a dimethylamino group, an ethylamino group, a diethylamino group, an n-propylamino group, a di-n-propylamino group, an isopropylamino group, a diisopropylamino group, an n-butylamino group, a di-n-butylamino group, a sec-butylamino group, a di-sec-butylamino group, a tert-butylamino group, a di-tert-butylamino group, an n-pentylamino group, a 2,2-dimethylpropylamino group, an n-hexylamino group, a cyclohexylamino group, an n-heptylamino group, an n-octylamino group, an n-nonylamino group, an n-decylamino group, an n-undecylamino group, an n-dodecylamino group, an n-tridecylamino group, an n-tetradecylamino group, an n-pentadecylamino group, an n-hexadecylamino group, an n-heptadecylamino group, an n-octadecylamino group, an n-nonadecylamino group, an n-icosyl amino group, a pyrrolyl group, a pyrrolidinyl group, a piperidinyl group, a carbazolyl group, a dihydroindolyl group and a dihydroisoindolyl group.

As (a1), a C1-C20 unsubstituted alkyl group, a C1-C20 alkyl group substituted with one or two or more fluorine atoms such as a trifluoromethyl group, a C1-C20 alkyl group substituted with a C1-C20 alkoxy group such as a methoxymethyl group and a C1-C20 alkyl group substituted with a cyano group such as a cyanomethyl group are preferable.

As (b1), a C1-C20 unsubstituted alkoxy group and a C1-C20 alkoxy group substituted with a C1-C20 alkoxy group such as a methoxymethoxy group are preferable.

As (c1), a C6-C20 unsubstituted aryl group is preferable.

As (d1), a C6-C20 unsubstituted aryloxy group is preferable.

As (e1), a C2-C20 unsubstituted acyl group and a C2-C20 acyl group substituted with a C6-C20 aryloxy group such as a phenoxybenzoyl group are preferable.

As (f1), a C2-C20 unsubstituted acyloxy group and a C2-C20 acyloxy group substituted with a C6-C20 aryloxy group such as a phenoxybenzoyloxy group are preferable.

As (g1), a C6-C20 unsubstituted arylsulfonyl group is preferable.

As (h1), a group wherein A¹ is an isopropoxy group, a 2,2-dimethypropoxy group, a cyclohexyloxy group, a diethylamino group or an n-dodecylamino group is preferable and a group wherein A¹ is an isopropoxy group, a 2,2-dimethylpropoxy group or a cyclohexyloxy group is more preferable.

As the group uninvolved in the reaction, the above-mentioned (a1), (b1), (e1) and (h1) are preferable.

Examples of the leaving group include a chlorine atom, a bromine atom, an iodine atom, a C1-C6 alkylsulfonyloxy group such as a trifluoromethylsulfonyloxy group, a methanesulfonyloxy group and an ethylsulfonyloxy group, and a C6-C10 arylsulfonyloxy group such as a phenylsulfonyloxy group and a p-methylphenylsulfonyloxy group, and a chlorine atom, a bromine atom and an iodine atom are preferable and a chlorine atom and a bromine atom are more preferable.

Specific examples of the aromatic compound include an aromatic compound represented by the formula (2):

Ar^{1_}(X¹)ₙ (2)

(hereinafter, simply referred to as the aromatic compound (2)).

In the formula (2), Ar¹ represents an n-valent aromatic group, and the aromatic ring of which the above-mentioned aromatic group is composed is a benzene ring, a biphenyl ring, a naphthalene ring, a fluorene ring, an anthracene ring, a phenanthrene ring, a thiophene ring, a pyrrole ring, a pyridine ring, a pyrimidine ring, a quinoline ring, an isoquinoline ring or a quinoxaline ring, and is an aromatic ring which may be substituted with at least one group uninvolved in the reaction. Additionally, X¹ represents a leaving group, and n represents 1 or 2. When n is 2, X¹s may be same or different each other.

Examples of the group uninvolved in the reaction include the same as described above.

Examples of the leaving group include the same as described above, and a chlorine atom, a bromine atom and an iodine atom are preferable and a chlorine atom and a bromine atom are more preferable.

Examples of the aromatic compound (2) include chlorobenzene, bromobenzene, iodobenzene, 4-chloro-1-fluorobenzene, 3-chloro-1-fluorobenzene, 2-chloro-1-fluorobenzene, 2-chlorotoluene, 2,5-dimethylchlorobenzene, 2-ethylchlorobenzene, 3-n-propylchlorobenzene, 4-isopropylchlorobenzene, 5-n-butylchlorobenzene, 2-isobutylchlorobenzene, 3-sec-butylchlorobenzene, 4-tert-butylchlorobenzene, 3-neo-pentylchlorobenzene, 2-n-hexylchlorobenzene, 4-cyclohexylchlorobenzene, 4-benzylchlorobenzene, 4-chlorobenzonitrile, 4-chlorobiphenyl, 2-chlorobiphenyl, 4-chlorobenzotrifluoride, 2-chlorobenzotrifluoride, (4-chlorophenyl)acetonitrile, 3-chloroanisole, 4-chloroanisole, 2,3-dimethoxychlorobenzene, 2,4-dimethoxychlorobenzene, 2,5-dimethoxychlorobenzene, 2-ethoxychlorobenzene, 3-n-propoxychlorobenzene, 4-isopropoxychlorobenzene, 5-n-butoxychlorobenzene, 4-tert-butoxychlorobenzene, 4-phenoxychlorobenzene, 4-benzyloxychlorobenzene, 4-(methoxymethyl)chlorobenzene, 4-(n-butoxymethyl)chlorobenzene, 4-(methoxymethoxy)chlorobenzene, 4-(benzyloxymethoxy)chlorobenzene, 4-{2-(n-butoxy)ethoxylchlorobenzene, 4-chloroacetophenone, 2-chloroacetophenone, 4-chloropropiophenone, 1-(4-chlorophenyl)-2,2-dimethylpropanone, (4-chlorobenzoyl)cyclohexane, 4-chlorobenzophenone, p-chlorobenzalacetone, 1-(4-chlorophenyl)-3-phenylpropen-1-one, 3-(4-chlorophenyl)-1-phenylpropen-1-one, 1-chloro-4-(phenylsulfonyl)benzene, 4-chlorophenyl p-tolyl sulfone,
methyl 4-chlorobenzoate, methyl 2-chlorobenzoate, ethyl 3-chlorobenzoate, n-propyl 4-chlorobenzoate, n-butyl 3-chlorobenzoate, 2,2-dimethylpropyl 2-chlorobenzoate, phenyl 4-chlorobenzoate, methyl p-chlorophenylacetate, methyl 3-(4-chlorophenyl)propionate, methyl p-chlorocinnamate, 4-chlorophenyl acetate, 2-chlorophenyl acetate, 4-chlorophenyl propionate, 4-chlorophenyl pivalate, 4-(tert-butoxycarbonyloxy)chlorobenzene, 4-chlorobenzyl acetate, (4-chlorophenyl) methyl sulfoxide, (4-chlorophenyl) phenyl sulfoxide, (4-chlorophenyl) ethyl sulfone, methyl 4-chlorobenzenesulfonate, methyl 3-chlorobenzenesulfonate, methyl 2-chlorobenzenesulfonate, ethyl 4-chlorobenzenesulfonate, 2,2-dimethylpropyl 4-chlorobenzenesulfonate, 2,2-dimethylpropyl 3-chlorobenzenesulfonate, 2,2-dimethylpropyl 2-chlorobenzenesulfonate, N,N-dimethyl-4-chlorobenzenesulfonamide, N,N-dimethyl-3-chlorobenzenesulfonamide, N,N-dimethyl-2-chlorobenzenesulfonamide, N,N-diethyl-4-chlorobenzenesulfonamide,
1-chloronaphthalene, 2-bromothiophene, 5-bromo-3-hexylthiophene, 2-bromo-3-dodecylthiophene, 5-bromo-2,2'-bithiophene, 5-bromo-3-cyclohexylthiophene, 2-chloro-3-octylthiophene, 5-chloro-3-phenylthiophene, 1-methyl-5-chloropyrrole, 1-hexyl-2-bromopyrrole, 1-octyl-5-chloropyrrole, 2-chloropyridine, 3-chloropyridine, 5-bromopyridine, 3-methyl-2-chloropyridine, 3-hexyl-5-chloropyridine, 5-chloro-2,2'-bipyridine, 3,3'-dimethyl-5-chloro-2,2'-bipyridine,
3,3'-dioctyl-5-bromo-2,2'-bipyridine, 2-chloropyrimidine, 5-chloropyrimidine, 2-bromopyrimidine, 5-chloroquinoline, 8-bromoquinoline, 2-chloroquinoline, 1-chloroisoquinoline, 4-chloroisoquinoline, 8-bromoisoquinoline, 5-bromoisoquinoline, 4-bromo-2,1,3-benzothiadiazole, 7-chlorobenzimidazole, 4-chlorobenzimidazole, 5-chloroquinoxaline, 5-chloro-2,3-diphenylquinoxaline, 2-bromoquinoxaline, 6-bromoquinoxaline, 1,3-dichlorobenzene, 1,4-dibromobenzene, 1,4-diiodobenzene, 2,4-dichlorotoluene, 3,5-dibromotoluene, 2,5-diiodotoluene, 1,3-dichloro-4-methoxybenzene, 1,4-dibromo-3-methoxybenzene, 1,4-diiodo-3-methoxybenzene, 1,3-dichloro-4-acetoxybenzene, 1,4-dibromo-3-acetoxybenzene, 1,3-diiodo-4-acetoxybenzene, 2,5-dichloro-4'-phenoxybenzophenone, 1,4-dibromo-2-ethylbenzene, 1,4-dibromo-2-methoxybenzene, dimethyl 2,5-dibromoterephthalate, 1,4-dibromonaphthalene, 1,1'-dibromo-4,4'-biphenyl, 1,4-dibromo-2,5-dihexyloxybenzene, 1-bromo-4-chlorobenzene, 1-bromo-4-chlorotoluene, 1-bromo-4-chloro-2-propylbenzene, 2,5-dibromo-4'-phenoxybenzophenone, 2,5-dibromothiophene, 2,5-dibromo-3-hexylthiophene, 2,5-dibromo-3-dodecylthiophene, 5,5'-dibromo-2,2'-bithiophene, 2,5-dibromo-3-cyclohexylthiophene, 2,5-dichloro-3-octylthiophene, 2,5-dichloro-3-phenylthiophene, 1-methyl-2,5-dichloropyrrole, 1-hexyl-2,5-dibromopyrrole, 1-octyl-2,5-dichloropyrrole, 2,5-dichloropyridine, 3,5-dichloropyridine, 2,5-dibromopyridine, 3-methyl-2,5-dichloropyridine, 3-hexyl-2,5-dichloropyridine, 5,5'-dichloro-2,2'-bipyridine, 3,3'-dimethyl-5,5'-dichloro-2,2'-bipyridine, 3,3'-dioctyl-5,5'-dibromo-2,2'-bipyridine, 2,5-dichloropyrimidine, 2,5-dibromopyrimidine, 5,8-dichloroquinoline, 5,8-dibromoquinoline, 2,6-dichloroquinoline, 1,4-dichloroisoquinoline, 5,8-dibromoisoquinoline, 4,7-dibromo-2,1,3-benzothiadiazole, 4,7-dichlorobenzimidazole, 5,8-dichloroquinoxaline, 5,8-dichloro-2,3-diphenylquinoxaline, 2,6-dibromoquinoxaline, 2,7-dibromo-9,9-dihexyl-9H-fluorene, 2,7-dibromo-9,9-dioctyl-9H-fluorene, 2,7-dibromo-9,9-didodecyl-9H-fluorene, 2,7-dichloro-9,9-dihexyl-9H-fluorene, 2,7-dichloro-9,9-dioctyl-9H-fluorene, 2,7-dichloro-9,9-didodecyl-9H-fluorene, 2-bromo-7-chloro-9,9-dihexyl-9H-fluorene, 2-bromo-7-chloro-9,9-dioctyl-9H-fluorene and 2-bromo-7-chloro-9,9-didodecyl-9H-fluorene.

As the aromatic compound (2), a commercially available one may be used and one produced according to known methods may be used.

Specific examples of the aromatic compound also include an aromatic compound represented by the formula (3): 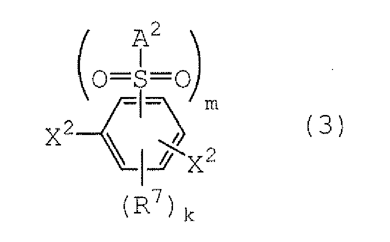 wherein A² represents an amino group substituted with one or two C1-C20 hydrocarbon groups, or a C1-C20 alkoxy group, and the above-mentioned hydrocarbon and alkoxy groups may be substituted with at least one group selected from the group consisting of a fluorine atom, a C1-C20 alkoxy group, a C6-C20 aryl group, a C6-C20 aryloxy group, a C2-C20 acyl group and a cyano group, R⁷ is independently in each occurrence a hydrogen atom, a fluorine atom, a C1-C20 alkyl group, a C1-C20 alkoxy group, a C6-C20 aryl group, a C6-C20 aryloxy group, a C2-C20 acyl group or a cyano group, and the above-mentioned C1-C20 alkyl, C1-C20 alkoxy, C6-C20 aryl, C6-C20 aryloxy and C2-C20 acyl groups may be substituted with at least one substituent selected from the group consisting of a fluorine atom, a cyano group, a C1-C20 alkoxy group, a C6-C20 aryl group and a C6-C20 aryloxy group, and the neighboring two R⁷s may be bonded to form a ring,
x² represents a chlorine atom, a bromine atom or an iodine atom, and m represents 1 or 2 and k represents 4-m (hereinafter, simply referred to as the aromatic compound (3)).

Examples of A² include the same as the above-mentioned A¹, and a C3-C20 unsubstituted alkoxy group is preferable and an isopropyl group, an isobutoxy group, a 2,2-dimethylpropoxy group and a cyclohexyloxy group are more preferable.

Examples of the C1-C20 alkyl group, the C1-C20 alkoxy group, the C6-C20 aryl group, the C6-C20 aryloxy group and the C2-C20 acyl group in R⁷ include the same as described above, respectively. As R⁷, a hydrogen atom, a C1-C20 unsubstituted alkyl group and a C1-C20 unsubstituted alkoxy group are preferable.

As X², a chlorine atom and a bromine atom are preferable and m is preferably 1.

Specific examples of the aromatic compound (3) include isopropyl 2,5-dichlorobenzenesulfonate, isobutyl 2,5-dichlorobenzenesulfonate, 2,2-dimethylpropyl 2,5-dichlorobenzenesulfonate, cyclohexyl 2,5-dichlorobenzenesulfonate, n-octyl 2,5-dichlorobenzenesulfonate, n-pentadecyl 2,5-dichlorobenzenesulfonate, n-icosyl 2,5-dichlorobenzenesulfonate, N,N-diethyl-2,5-dichlorobenzenesulfonamide, N,N-diisapropyl-2,5-dichlorobenzenesulfonamide, N-(2,2-dimethylpropyl)-2,5-dichlorobenzenesulfonamide, N-n-dodecyl-2,5-dichlorobenzenesulfonamide,
N-n-icosyl-2,5-dichlorobenzenesulfonamide, isopropyl 3,5-dichlorobenzenesulfonate, isobutyl 3,5-dichlorobenzenesulfonate, 2,2-dimethylpropyl 3,5-dichlorobenzenesulfonate, cyclohexyl 3,5-dichlorobenzenesulfonate, n-octyl 3,5-dichlorobenzenesulfonate, n-pentadecyl 3,5-dichlorobenzenesulfonate, n-icosyl 3,5-dichlorobenzenesulfonate, N,N-diethyl-3,5-dichlorobenzenesulfonamide, N,N-diisopropyl-3,5-dichlorobenzenesulfonamide, N-(2,2-dimethylpropyl)-3,5-dichlorobenzenesulfonamide, N-n-dodecyl-3,5-dichlorobenzenesulfonamide, N-n-icosyl-3,5-dichlorobenzenesulfonamide, isopropyl 2,5-dibromobenzenesulfonate, isobutyl 2,5-dibromobenzenesulfonate, 2,2-dimethylpropyl 2,5-dibromobenzenesulfonate, cyclohexyl 2,5-dibromobenzenesulfonate, n-octyl 2,5-dibromobenzenesulfonate, n-pentadecyl 2,5-dibromobenzenesulfonate, n-icosyl 2,5-dibromobenzenesulfonate, N,N-diethyl-2,5-dibromobenzenesulfonamide, N,N-diisopropyl-2,5-dibromobenzenesulfonamide, N-(2,2-dimethylpropyl)-2,5-dibromobenzenesulfonamide, N-n-dodecyl-2,5-dibromobenzenesulfonamide, N-n-icosyl-2,5-dibromobenzenesulfonamide, isopropyl 3,5-dibromobenzenesulfonate, isobutyl 3,5-dibromobenzenesulfonate, 2,2-dimethylpropyl 3,5-dibromobenzenesulfonate, cyclohexyl 3,5-dibromobenzenesulfonate, n-octyl 3,5-dibromobenzenzenesulfonate, n-pentadecyl 3,5-dibromobenzenesulfonate, n-icosyl 3,5-dibromobenzenesulfonate, N,N-diethyl-3,5-dibromobenzenesulfonamide, N,N-diisopropyl-3,5-dibromobenzenesulfonamide, N-(2,2-dimethylpropyl)-3,5-dibromobenzenesulfonamide, N-n-dodecyl-3,5-dibromobenzenesulfonamide, N-n-icosyl-3,5-dibromobenzenesulfonamide, isopropyl 2,5-diiodobenzenesulfonate, isobutyl 2,5-diiodobenzenesulfonate, 2,2-dimethylpropyl 2,5-diiodobenzenesulfonate, cyclohexyl 2,5-diiodobenzenesulfonate, n-octyl 2,5-diiodobenzenesulfonate, n-pentadecyl 2,5-diiodobenzenesulfonate, n-icosyl 2,5-diiodobenzenesulfonate, N,N-diethyl-2,5-diiodobenzenesulfonamide, N,N-diisopropyl-2,5-diiodobenzenesulfonamide, N-(2,2-dimethylpropyl)-2,5-diiodobenzenesulfonamide, N-n-dodecyl-2,5-diiodobenzenesulfonamide, N-n-icosyl-2,5-diiodobenzenesulfonamide, isopropyl 3,5-diiodobenzenesulfonate, isobutyl 3,5-diiodobenzenesulfonate, 2,2-dimethylpropyl 3,5-diiodobenzenesulfonate, cyclohexyl 3,5-diiodobenzenesulfonate, n-octyl 3,5-diiodobenzenesulfonate, n-pentadecyl 3,5-diiodobenzenesulfonate, n-icosyl 3,5-diiodobenzenesulfonate, N,N-diethyl-3,5-diiodobenzenesulfonamide, N,N-diisopropyl-3,5-diiodobenzenesulfonamide, N-(2,2-dimethylpropyl)-3,5-diiodobenzenesulfonamide, N-n-dodecyl-3,5-diiodobenzenesulfonamide, N-n-icosyl-3,5-diiodobenzenesulfonamide, 2,2-dimethylpropyl 2,4-dichlorobenzenesulfonate, 2,2-dimethylpropyl 2,4-dibromobenzenesulfonate, 2,2-dimethylpropyl 2,4-diiodobenzenesulfonate, 2,2-dimethylpropyl 2,4-dichloro-5-methylbenzenesulfonate, 2,2-dimethylpropyl 2,5-dichloro-4-methylbenzenesulfonate, 2,2-dimethylpropyl 2,4-dibromo-5-methylbenzenesulfonate, 2,2-dimethylpropyl 2,5-dibromo-4-methylbenzenesulfonate, 2,2-dimethylpropyl 2,4-diiodo-5-methylbenzenesulfonate, 2,2-dimethylpropyl 2,5-diiodo-4-methylbenzenesulfonate, 2,2-dimethylpropyl 2,4-dichloro-5-methoxybenzenesulfonate, 2,2-dimethylpropyl 2,5-dichloro-4-methoxybenzenesulfonate, 2,2-dimethylpropyl 2,4-dibromo-5-methoxybenzenesulfonate, 2,2-dimethylpropyl 2,5-dibromo-4-methoxybenzenesulfonate, 2,2-dimethylpropyl 2,4-diiodo-5-methoxybenzenesulfonate, 2,2-dimethylpropyl 2,5-diiodo-4-methoxybenzenesulfonate and 1-(2,5-dichlorobenzenesulfonyl)pyrrolidine.

Among them, 2,2-dimethylpropyl 2,5-dichlorobenzenesulfonate, isobutyl 2,5-dichlorobenzenesulfonate, cyclohexyl 2,5-dichlorobenzenesulfonate, N,N-diethyl-2,5-dichlorobenzenesulfonamide and N-n-dodecyl-2,5-dichlorobenzenesulfonamide, 2,2-dimethylpropyl 2,5-dibromobenzenesulfonate, isobutyl 2,5-dibromobenzenesulfonate, cyclohexyl 2,5-dibromobenzenesulfonate, N,N-diethyl-2,5-dibromobenzenesulfonamide and N-n-dodecyl-2,5-dibromobenzenesulfonamide are preferable.

Specific examples of the aromatic compound also include an aromatic compound represented by the formula (4): 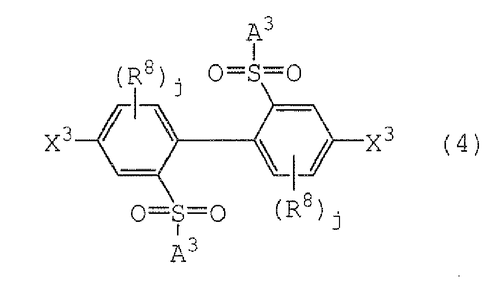 wherein A³ represents an amino group substituted with one or two C1-C20 hydrocarbon groups, or a C3-C20 alkoxy group, and the above-mentioned hydrocarbon and alkoxy groups may be substituted with at least one group selected from the group consisting of a fluorine atom, a C1-C20 alkoxy group, a C6-C20 aryl group, a C6-C20 aryloxy group, a C2-C20 acyl group and a cyano group, R⁸ is independently in each occurrence a fluorine atom, a C1-C20 alkyl group, a C1-C20 alkoxy group, a C6-C20 aryl group, a C6-C20 aryloxy group, a C2-C20 acyl group or a cyano group, and the above-mentioned C1-C20 alkyl, C1-C20 alkoxy, C6-C20 aryl, C6-C20 aryloxy and C2-C20 acyl groups may be substituted with at least one substituent selected from the group consisting of a fluorine atom, a cyano group, a C1-C20 alkoxy group, a C6-C20 aryl group and a C6-C20 aryloxy group, and the neighboring two R⁸s may be bonded to form a ring,
X³ represents a chlorine atom, a bromine atom or an iodine atom, and j represents an integer of 0 to 3 (hereinafter, simply referred to as the atomatic compound (4)).

Examples of A³ include the same as the above-mentioned A², and a C3-C20 unsubstituted alkoxy group is preferable, and an isopropyl group, an isobutoxy group, a 2, 2-dimethylpropoxy group and a cyclohexyloxy group are more preferable.

Examples of the C1-C20 alkyl group, the C1-C20 alkoxy group, the C6-C20 aryl group, the C6-C20 aryloxy group and the C2-C20 acyl group in R⁸ include the same as described above, respectively. As R⁸, a C1-C20 unsubstituted alkyl group and a C1-C20 unsubstituted alkoxy group are preferable.

As X³, a chlorine atom and a bromine atom are preferable and j is preferably 0.

Examples of the aromatic compound (4) include dimethyl 4,4'-dichlorobiphenyl-2,2'-disulfonate, diethyl 4,4'-dichlorobiphenyl-2,2'-disulfonate, di(n-propyl) 4,4'-dichlorobiphenyl-2,2'-disulfonate, diisopropyl 4,4'-dichlorobiphenyl-2,2'-disulfonate, di(n-butyl) 4,4'-dichlorobiphenyl-2,2'-disulfonate, diisobutyl 4,4'dichlorobiphenyl-2,2'-disulfonate, di(2,2-dimethylpropyl) 4,4'-dichlorobiphenyl-2,2'-disulfonate, dicyclohexyl 4,4'-dichlorobiphenyl-2,2'-disulfonate, di (n-octyl) 4,4'-dichlorobiphenyl-2,2'-disulfonate, di(n-pentadecyl) 4,4'-dichlorobiphenyl-2,2'-disulfonate, di(n-icosyl) 4,4'-dichlorobiphenyl-2,2'-disulfonate, N,N-dimethyl-4,4'-dichlorobiphenyl-2,2'-disulfonamide, N,N-diethyl-4,4'-dichlorobiphenyl-2,2'-disulfonamide, N,N-di(n-propyl)-4,4'-dichlorobiphenyl-2,2'-disulfonamide, N,N-diisopropyl-4,4'-dichlorobiphenyl-2,2'-disulfonamide, N,N-di(n-butyl)-4,4'-dichlorobiphenyl-2,2'-disulfonamide, N,N-diisobutyl-4,4'-dichlorobiphenyl-2,2'-disulfonamide, N-di(2,2-dimethylpropyl)-4,4'-dichlorobiphenyl-2,2'-disulfon amide, N-di(n-octyl)-4,4'-dichlorobiphenyl-2,2'-disulfonamide, N-di(n-dodecyl)-4,4'-dichlorobiphenyl-2,2'-disulfonamide, N,N-di(n-icosyl)-4,4'-dichlorobipienyl-2,2'-disulfonamide, N,N-diphenyl-4,4'-dichlorobiphenyl-2,2'-disulfonamide, di(2,2-dimethylpropyl) 3,3'-dimethyl-4,4'-dichlorobiphenyl-2,2'-disulfonate, di(2,2-dimethylpropyl) 5,5'-dimethyl-4,4'-dichlorobiphenyl-2,2'-disulfonate, di(2,2-dimethylpropyl) 6,6'-dimethyl-4,4'-dichlorobiphenyl-2,2'-disulfonate, di(2,2-dimethylpropyl) 3,3'-dimethoxy-4,4'-dichlorobiphenyl-2,2'-disulfonate, di(2,2-dimethylpropyl) 5,5'-dimethoxy-4,4'-dichlorobiphenyl-2,2'-disulfonate, di(2,2-dimethylpropyl) 6,6'-dimethoxy-4,4'-dichlorobiphenyl-2,2'-disulfonate, di(2,2-dimethylpropyl) 3,3'-diphenyl-4,4'-dichlorobiphertyl-2,2'-disulfonate, di(2,2-dimethylpropyl) 3,3'-diacetyl-4,4'-dichlorobiphenyl-2,2'-disulfonate, di(2,2-dimethylpropyl) 5,5'-diacetyl-4,4'-dichlorobiphenyl-2,2'-disulfonate, dimethyl 4,4'-dibromobiphenyl-2,2'-disulfonate, diethyl 4,4'-dibromobiphenyl-2,2'-disulfonate, di(n-propyl) 4,4'-dibromobiphenyl-2,2'-disulfonate, diisopropyl 4,4'-dibromobiphenyl-2,2'-disulforiate, di(n-butyl) 4,4'-dibromobiphenyl-2,2'-disulfonate, diisobutyl 4,4'-dibromobiphenyl-2,2'-disulfonate, di(2,2-dimethylpropyl) 4,4'-dibromobiphenyl-2,2'-disulfonate, dicyclohexyl 4,4'-dibromobiphenyl-2,2'-disulfonate, di(n-octyl) 4,4'-dibromobiphenyl-2,2'-disulfonate, di(n-pentadecyl) 4,4'-dibromobiphenyl-2,2'-disulfonate, di(n-icosyl) 4,4'-dibromobiphenyl-2,2'-disulfonate, N,N-dimethyl-4,4'-dibromobiphenyl-2,2'-disulfonamide, N,N-diethyl-4,4'-dibromobiphenyl-2,2'-disulfonamide, N,N-di(n-propyl)-4,4'-dibromabiphenyl-2,2'-disulfonamide, N,N-diisopropyl-4,4'-dibromobiphenyl-2,2'-disulfonamide, N,N-di(n-butyl)-4,4'-dibromobiphenyl-2,2'-disulfonamide, N,N-diisobutyl-4,4'-dibromobiplienyl-2,2'-disulfonamide, N-di(2,2-dimethylpropyl)-4,4'-dibromobiphenyl-2,2'-disulfona mide, N-di(n-octyl)-4,4'-dibromobiphenyl-2,2'-disulfonamide, N-di(n-dodecyl)-4,4'-dibromobiphenyl-2,2'-disulfonamide, N-di(n-icosyl)-4,4'-dibromobiphenyl-2,2'-disulfonamide and N,N-diphenyl-4,4'-dibromobiphenyl-2,2'-disulfonamide.

Among them, diisopropyl 4,4'-dichlorobiphenyl-2,2'-disulfonate, di(2,2-dimethylpropyl) 4,4'-dichlorobiphenyl-2,2'-disulfonate, diisopropyl 4,4'-dibromobiphenyl-2,2'-disulfonate and di(2,2-dimethylpropyl) 4,4'-dibrormobiphenyl-2,2'-disulfonate are preferable.

The aromatic compound (3) can be produced, for example, according to the method described in WO2007/043274.

The aromatic compound (4) can be produced, for example, according to the method described in WO2007/102235.

Specific examples of the aromatic compound also include an aromatic compound represented by the formula (5): 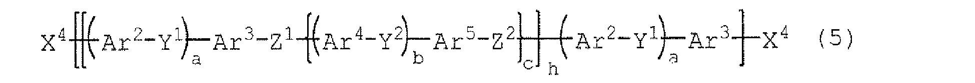 wherein a, b and c each independently represent 0 or 1, and h represents an integer of 5 or more,
Ar², Ar³, Ar⁴ and Ar⁵ are the same or different and represent a divalent aromatic group, and the divalent aromatic group may be substituted with at least one substituent selected from the group consisting of the following (a2) to (e2):
(a2) a C1-C20 alkyl group which may be substituted with at least one substituent selected from the group consisting of a fluorine atom, a cyano group, a C1-C20 alkoxy group, a C6-C20 aryl group and a C6-C20 aryloxy group;
(b2) a C1-C20 alkoxy group which may be substituted with at least one substituent selected from the group consisting of a fluorine atom, a cyano group, a C1-C20 alkoxy group, a C6-C20 aryl group and a C6-C20 aryloxy group;
(c2) a C6-C20 aryl group which may be substituted with at least one substituent selected from the group consisting of a fluorine atom, a cyano group, a C1-C20 alkoxy group and a C6-C10 aryloxy group;
(d2) a C6-C20 aryloxy group which may be substituted with at least one substituent selected from the group consisting of a fluorine atom, a cyano group, a C1-C20 alkoxy group and a C6-C20 aryloxy group; and
(e2) a C2-C20 acyl group which may be substituted with at least one substituent selected from the group consisting of a fluorine atom, a cyano group, a C1-C20 alkoxy group, a C6-C20 aryl group and a C6-C20 aryloxy group,
   Y¹ and Y² each independently represent a single bond, -CO-, -SO₂-, -C(CH₃)₂-, -C(CF₃)₂- or a fluorene-9,9-diyl group,
   Z¹ and Z² each independently represent -O- or -S-, and X⁴ represents a chlorine atom, a bromine atom or an iodine atom (hereinafter, simply referred to as the aromatic compound (5)).

In the formula (5), h is preferably an integer of 10 or more.

Examples of the divalent aromatic group in Ar², Ar³, Ar⁴ and Ar⁵ include a divalent monocyclic aromatic group such as a 1,3-phenylene group, a 1,4-phenylene group and 4,4'-biphenyl-1,1'-diyl group; a divalent condensed aromatic group such as a naphthalene-1,3-diyl group, a naphthalene-1,4-diyl group, a naphthalene-1,5-diyl group, a naphthalene-1,6-diyl group, a naphthalene-1,7-diyl group, a naphthalene-2,6-diyl group, a naphthalene-2,7-diyl group and a 9H-fluorene-2,7-diyl group; and a divalent heteroaromatic group such as a pyridine-2,5-diyl group, a pyridine-2,6-diyl group, a quinoxaline-2,6-diyl group, a thiophene-2,5-diyl group, 2,2'-bithiophene-5,5'-diyl group, a pyrrole-2,5-diyl group, a 2,2'-bipyridine-5,5'-diyl group, a pyrimidine-2,5-diyl group, a quinoline-5,8-diyl group, a quinoline-2,6-diyl group, an isoquinoline-1,4-diyl group, an isoquinoline-5,8-diyl group, 2,1,3-benzothiadiazole-4,7-diyl group, a benzimidazole-4,7-diyl group, a quinoxaline-5,8-diyl group and a quinoxaline-2,6-diyl group. Among them, the divalent monocyclic aromatic group and the divalent condensed aromatic group are preferable, and a 1,4-phenylene group, a naphthalene-1,4-diyl group, a naphthalene-1,5-diyl group, a naphthalene-2, 6-diyl group and a naphthalene-2,7-diyl group are more preferable.

The divalent aromatic group may be substituted with at least one substituent selected from the group consisting of the following (a2) to (e2).
(a2) a C1-C20 alkyl group which may be substituted with at least one substituent selected from the group consisting of a fluorine atom, a cyano group, a C1-C20 alkoxy group, a C6-C20 aryl group and a C6-C20 aryloxy group;
(b2) a C1-C20 alkoxy group which may be substituted with at least one substituent selected from the group consisting of a fluorine atom, a cyano group, a C1-C20 alkoxy group, a C6-C20 aryl group and a C6-C20 aryloxy group;
(c2) a C6-C20 aryl group which may be substituted with at least one substituent selected from the group consisting of a fluorine atom, a cyano group, a C1-C20 alkoxy group and a C6-C10 aryloxy group;
(d2) a C6-C20 aryloxy group which may be substituted with at least one substituent selected from the group consisting of a fluorine atom, a cyano group, a C1-C20 alkoxy group and a C6-C20 aryloxy group; and
(e2) a C2-C20 acyl group which may be substituted with at least one substituent selected from the group consisting of a fluorine atom, a cyano group, a C1-C20 alkoxy group, a C6-C20 aryl group and a C6-C20 aryloxy group.

Examples of the C1-C20 alkoxy group, the C6-C20 aryl group, the C6-C20 aryloxy group, the C1-C20 alkyl, group and the C2-C20 acyl group in (a2) to (e2) include the same as described above.

Examples of (a2) include the same as the above-mentioned (a1). Examples of (b2) include the same as the above-mentioned (b2). Examples of (c2) include the same as the above-mentioned (c1). Examples of (d2) include the same as the above-mentioned (d1). Examples of (e2) include the same as the above-mentioned (e1).

As X⁴, a chlorine atom and a bromine atom are preferable.

Specific examples of the aromatic compound (5) include the following compounds and compounds wherein both terminal chlorine atoms in the following compounds are replaced to bromine atoms. Additionally, in the following formulae, h represents the same meanings as the above.

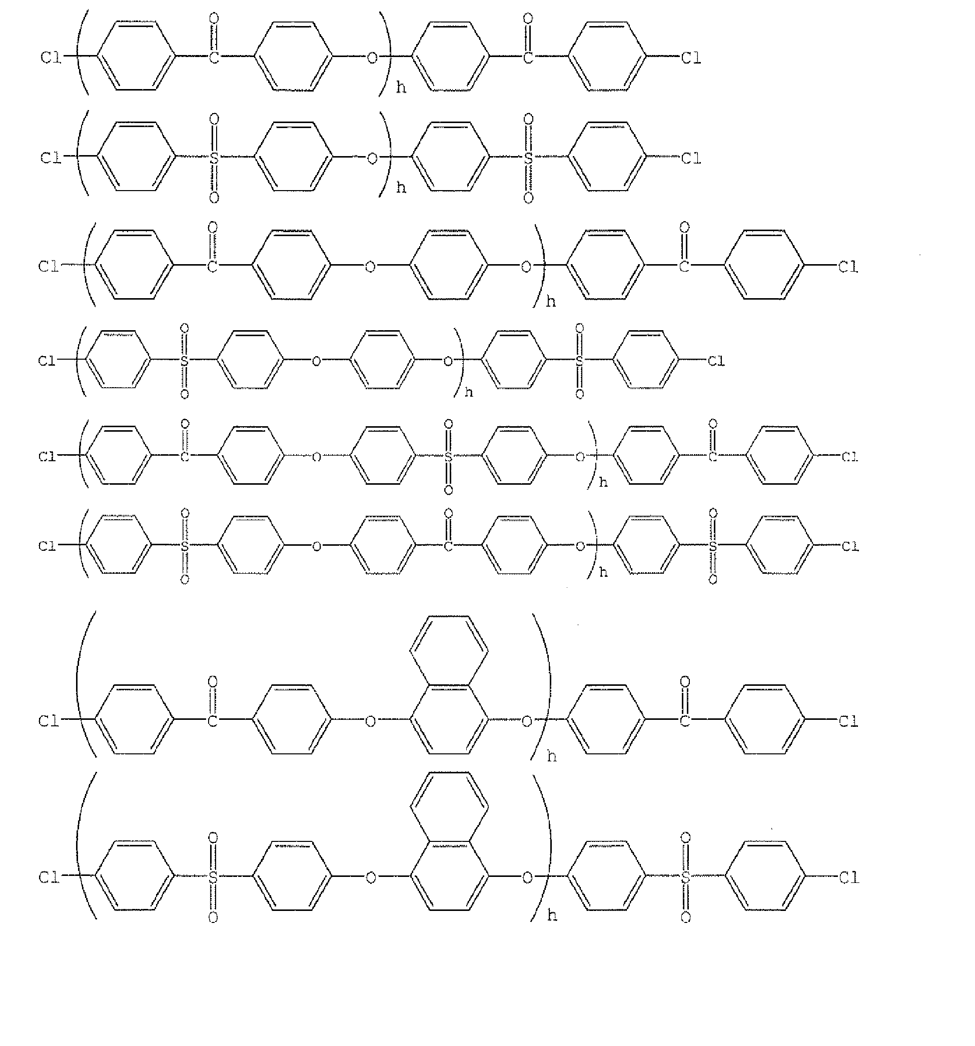

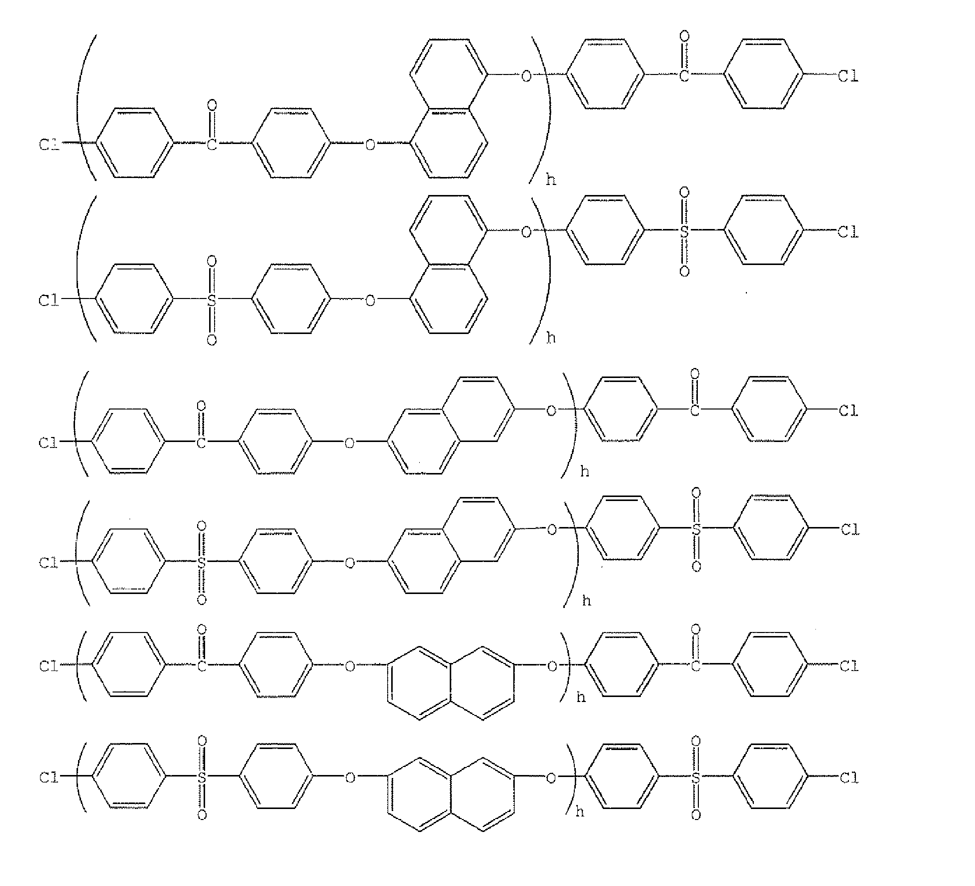

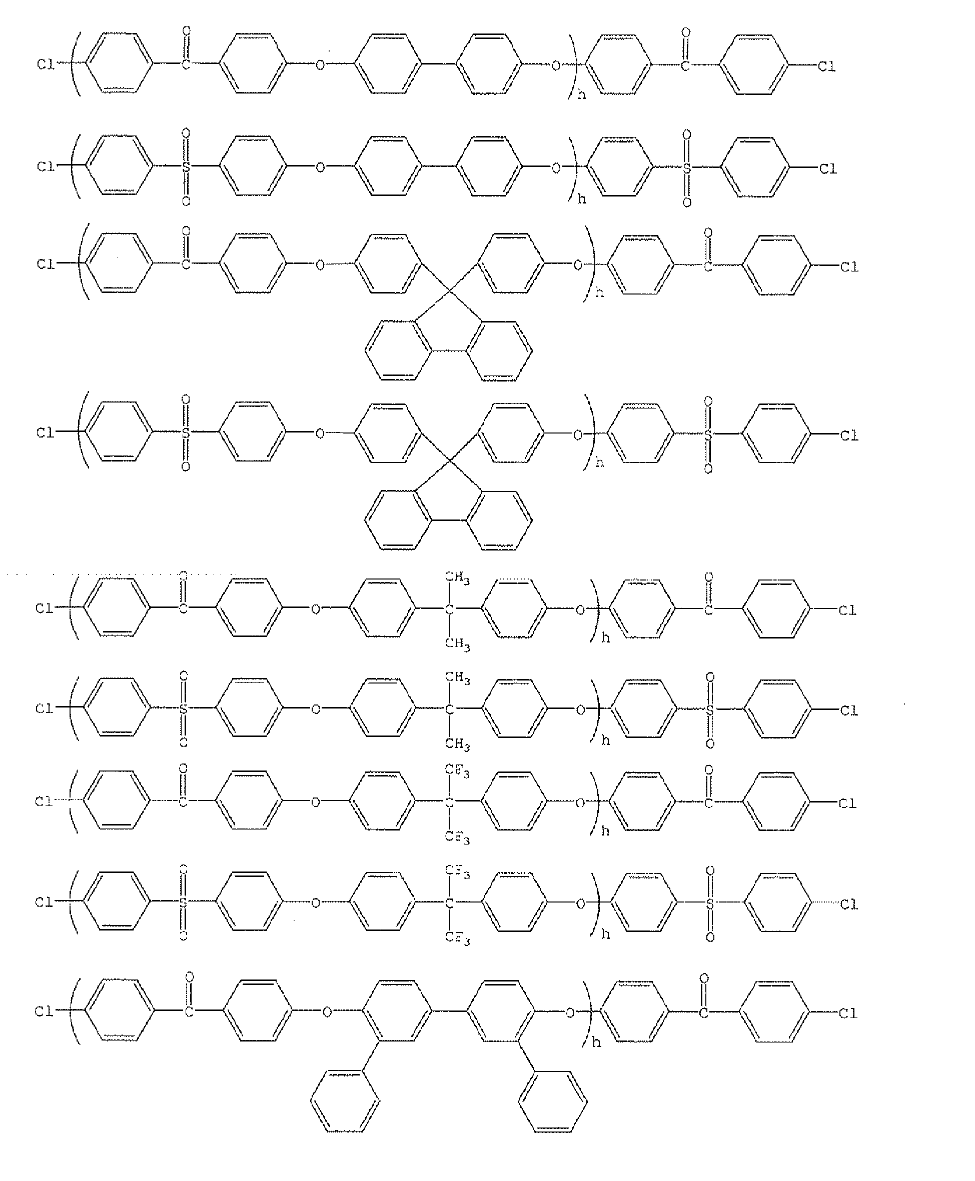

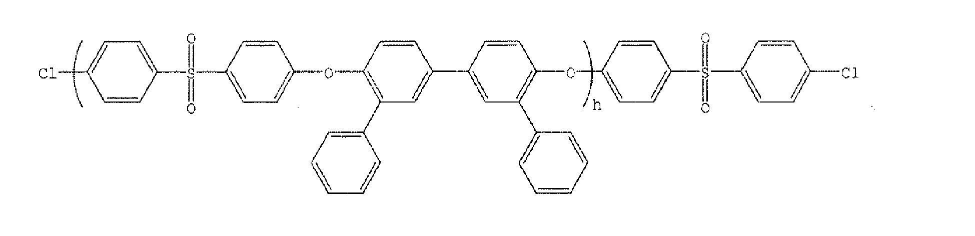

As the aromatic compound (5), one produced according to known methods such as JP Patent No. 2,745,727 may be used and a commercially available one may be used. Examples of the commercially available one include SUMIKA EXCEL PES manufactured by Sumitomo Chemical Company, Limited.

As the aromatic compound (5), one having 2,000 or more of weight average molecular weight equivalent to polystyrene is preferably used, and one having 3,000 or more of weight average molecular weight equivalent to polystyrene is more preferable.

The present invention is one comprising reacting an aromatic compound (A) with an aromatic compound (A) having the same structure as that of the above-mentioned aromatic compound (A) or an aromatic compound (B) being structurally different from the above-mentioned aromatic compound (A).

Specific examples of cases where an aromatic compound (A) is reacted with an aromatic compound (A) having the same structure as that of the above-mentioned aromatic compound (A) include
a case where the aromatic compound (2) is used as the aromatic compound (A);
a case where the aromatic compound (3) is used as the aromatic compound (A); and
a case where the aromatic compound (4) is used as the aromatic compound (A).

Specific examples of cases where an aromatic compound (A) is reacted with an aromatic compound (B) being structurally different from the above-mentioned aromatic compound (A) include a case where the aromatic compound (2) is used as the aromatic compound (A) and the aromatic compound (2) being structurally different from the above-mentioned aromatic compound (A) is used as the aromatic compound (B);
a case where the aromatic compound (2) is used as the aromatic compound (A) and the aromatic compound (3) being structurally different from the above-mentioned aromatic compound (A) is used as the aromatic compound (B);
a case where the aromatic compound (2) is used as the aromatic compound (A) and the aromatic compound (4) being structurally different from the above-mentioned aromatic compound (A) is used as the aromatic compound (B);
a case where the aromatic compound (2) is used as the aromatic compound (A) and the aromatic compound (5) being structurally different from the above-mentioned aromatic compound (A) is used as the aromatic compound (B);
a case where the aromatic compound (3) is used as the aromatic compound (A) and the aromatic compound (2) being structurally different from the above-mentioned aromatic compound (A) is used as the aromatic compound (B);
a case where the aromatic compound (3) is used as the aromatic compound (A) and the aromatic compound (3) being structurally different from the above-mentioned aromatic compound (A) is used as the aromatic compound (B);
a case where the aromatic compound (3) is used as the aromatic compound (A) and the aromatic compound (4) being structurally different from the above-mentioned aromatic compound (A) is used as the aromatic compound (B);
a case where the aromatic compound (3) is used as the aromatic compound (A) and the aromatic compound (5) being structurally different from the above-mentioned aromatic compound (A) is used as the aromatic compound (B);
a case where the aromatic compound (4) is used as the aromatic compound (A) and the aromatic compound (2) being structurally different from the above-mentioned aromatic compound (A) is used as the aromatic compound (B);
a case where the aromatic compound (4) is used as the aromatic compound (A) and the aromatic compound (3) being structurally different from the above-mentioned aromatic compound (A) is used as the aromatic compound (B);
a case where the aromatic compound (4) is used as the aromatic compound (A) and the aromatic compound (4) being structurally different from the above-mentioned aromatic compound (A) is used as the aromatic compound (B); and
a case where the aromatic compound (4) is used as the aromatic compound (A) and the aromatic compound (5) being structurally different from the above-mentioned aromatic compound (A) is used as the aromatic compound (B).

Examples of the nickel compound include a zero-valent nickel compound such as bis(cyclooctadiene)nickel(0) and tetrakis(triphenylphosphine)nickel(0); and a divalent nickel compound such as a nickel halide (for example, nickel fluoride, nickel chloride, nickel bromide, nickel iodide and the like), a nickel carboxylate (for example, nickel formate, nickel acetate and the like), nickel sulfate, nickel carbonate, nickel nitrate, nickel acetylacetonate and (dimethoxyethane)nickel chloride, and bis(cyclooctadiene)nickel(0) and nickel halide are preferable.

While the used amount of the nickel compound may be a catalytic amount, and when the used amount thereof is too small, a conjugated aromatic compound having a small molecular weight tends to be obtained, and when the used amount thereof is too much, the isolation of an conjugated aromatic compound after completion of reaction tends to be cumbersome, and therefore, the used amount of the nickel compound is usually 0.001 to 0.8 mole and preferably 0.01 to 0.3 mole per 1 mole of all of used aromatic compound.

The ligand is not limited in so far as it is capable of coordinating to nickel. Specific examples thereof include a ligand having a nitrogen atom or nitrogen atoms, a ligand having a phosphorus atom or phosphorus atoms, and a π-type ligand. Examples of the ligand having a nitrogen atom or nitrogen atoms include methylenebisoxazoline; N,N,N',N'-tetramethylethylenediamine; a 2,2'-bipyridine compound such as 2,2'-bipyridine, 4,4'-dimethyl-2,2'-bipyridine, 4,4'-diphenyl-2,2'-bipyridine and 4,4'-dimethoxy-2,2'-bipyridine; and a 1,10-phenanthroline compound such as 1,10-phenanthroline, 4,7-diphenyl-1,10-phenanthroline and 4,5,6,7-tetramethyl-1,10-phenanthroline.

Examples of the ligand having a phosphorus atom or phosphorus atoms include a triarylphosphine such as triphenylphosphine and tris(o-tolyl)phosphine; a trialkylphosphine such as tricyclohexylphosphine and tri-tert-butylphosphine, and a bidentate phosphorus ligand such as 1,1'-bis(di(4-trifluoromethylphenyl)phosphino)ferrocene and bis(2-diphenylphosphinophenyl) ether.

Examples of the π-type ligand include 1,5-cylooctadiene, trans-stilbene, 1,4-benzoquinone and 1,5-diphenyl-1,4-pentadien-3-one.

As the ligand, the ligand having a nitrogen atom or nitrogen atoms and the ligand having a phosphorus atom or phosphorus atoms are preferable, and the 2,2'-bipyridine compound, the 1,10-phenanthroline compound, the triarylphosphine and the bidentate phosphorus ligand are more preferable.

The used amount of the ligand is usually 0.5 mole or more per 1 mole of the nickel compound. While the upper limit thereof is not limited, when the used amount thereof is too much, the isolation of an conjugated aromatic compound after completion of reaction tends to be cumbersome and it is economically disadvantageous, and therefore, it is practically 0.5 to 10 moles and preferably 0.5 to 2 moles per 1 mole of the nickel compound.

The nickel complex wherein the ligand coordinates may be prepared by previously contacting the ligand with the nickel compound and the prepared nickel complex may be used as it is or after isolation.

"Metal reducing agent" means a metal capable of reducing divalent nickel to zero-valent nickel. Specific examples thereof include calcium, sodium, magnesium, aluminum, manganese, zinc and iron, and magnesium, manganese and zinc are preferable, and manganese and zinc are more preferable. As the metal reducing agent, commercially available one is usually used, and powdery or chip-type one is usually used. The used amount of the metal reducing agent is usually 1 mole or more per 1 mole of all of used aromatic compound. While the upper limit thereof is not limited, when the used amount thereof is too much, the isolation of an conjugated aromatic compound after completion of reaction tends to be cumbersome and it easily becomes to be economically disadvantageous, and therefore, it is practically 10 moles or less and preferably 5 moles or less.

Examples of the manganese salt include a manganese(II) halide such as manganese(II) fluoride, manganese(II) chloride, manganese(II) bromide and manganese(II) iodide; a manganese carboxylate such as manganese naphthenate, manganese (II) acetate, manganese(III) acetate and manganese(II) 2-ethyl-hexanoate;, manganese carbonyl; phthalocyanine manganese(II); manganese(II) bis(cyclopentadienyl); manganese(II) carbonate; manganese(II) nitrate; manganese(IV) oxide; manganese(II) oxide; manganese(II) sulfate; manganese(III) acetylacetonate; and manganese(II) acetylacetonate, and a divalent manganese salt is preferable and manganese(II) halide is more preferable.

The used amount of the manganese salt is usually 0.5 mole or more per 1 mole of nickel. While the upper limit thereof is not limited, when the used amount thereof is too much, the post-treatment after the coupling reaction tends to be cumbersome and it becomes to be economically disadvantageous, and therefore, it is practically 10 moles or less and preferably 5 moles or less.

The reaction of the aromatic compound (A) with the aromatic compound (A) having the same structure as that of the above-mentioned aromatic compound (A) or the aromatic compound (B) being structurally different from the above-mentioned aromatic compound (A) is usually carried out in the presence of a solvent. The solvent may be one in which the used aromatic compounds and the produced conjugated aromatic compound can be dissolved. Specific examples of the solvent include an aromatic hydrocarbon solvent such as toluene and xylene; an ether solvent such as tetrahydrofuran and 1,4-dioxane; an aprotic polar solvent such as dimethylsulfoxide, N-methyl-2-pyrrolidone, N,N-dimethylformamide, N,N-dimethylacetamide and hexamethylphosphoric triamide; and a halogenated hydrocarbon solvent such as dichloromethane and dichloroethane. These solvents may be used alone, and two or more kinds thereof may be mixed to use. Among them, the ether solvent and the aprotic polar solvent are preferable and tetrahydrofuran, dimethylsulfoxide, N-methyl-2-pyrrolidone and N,N-dimethylacetamide are more preferable. When the used amount of the solvent is too much, a conjugated aromatic compound having small molecular weight tends to be obtained, and when the used amount thereof is too small, the property of the reaction mixture tends to be bad, and therefore, it is usually 1 to 200 parts by weight and preferably 5 to 100 parts by weight per 1 part by weight of all of the aromatic compounds used.

The reaction is usually conducted by mixing the aromatic compounds, the divalent nickel compound, zinc and the phenathroline compound (1) in an atmosphere of an inert gas such as nitrogen gas. The reaction temperature is usually 0 to 250°C and preferably 30 to 100°C. The reaction time is usually 0.5 to 48 hours.

The conjugated aromatic compound can be obtained by thus reaction, and "conjugated aromatic compound" means a compound having at least one aromatic ring and possessing a delocated π-electron system in a part of or all of its molecule.

When the produced conjugated aromatic compound is a polymer, for example, after completion of the reaction, the conjugated aromatic compound is precipitated by mixing a solvent in which the produced conjugated aromatic compound is not soluble or is poorly soluble with the reaction mixture, followed by separating the precipitated conjugated aromatic compound from the reaction mixture by filtration, thereby being able to isolate it. The solvent in which the produced conjugated aromatic compound is not soluble or is poorly soluble is mixed with the reaction mixture and then adding an aqueous acid solution such as hydrochloric acid thereto and the precipitated conjugated aromatic compound may be separated from the reaction mixture by filtration. The molecular weight and structure of the obtained conjugated aromatic compound can be analyzed by a conventional means such as gel permeation chromatography and NMR. Examples of the solvent in which the produced conjugated aromatic compound is not soluble or is poorly soluble include water, methanol, ethanol and acetonitrile, and water and methanol are preferable.

When the produced conjugated aromatic compound is not a polymer, for example, after completion of the reaction, the produced conjugated aromatic compound can be isolated by concentrating the reaction mixture. The isolated conjugated aromatic compound may be further purified by a conventional purification means such as column chromatography, distillation and recrystallization.

Specific examples of the obtained conjugated aromatic compound are shown below.

In a case where an aromatic compound (A) is reacted with an aromatic compound (A) having the same structure as that of the above-mentioned aromatic compound (A) and the aromatic compound (2) wherein n is 1 is used as the aromatic compound (A), a conjugated aromatic compound represented by the following formula (20):

Ar^{1_}Ar¹ (20)

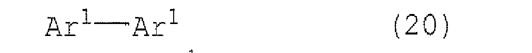

wherein Ar¹ is the same meaning as defined above, is obtained.

Examples of the conjugated aromatic compound represented by the formula (20) include biphenyl, 4,4'-difluorobiphenyl, 3,3'-difluorobiphenyl, 2,2'-difluorobiphenyl, 2,2'-dimethylbiphenyl, 2,2',5,5'-tetramethylbiphenyl, 2,2'-diethylbiphenyl, 3,3'-di-n-pronylbiphenyl, 4,4'-diisopropylbiphenyl, 5,5'-di-n-butylbiphenyl, 2,2'-diisobutylbiphenyl, 3,3'-di-sec-butylbiphenyl, 4,4'-di-tert-butylbiphenyl, 5,5'-bis(2,2-dimethylpropyl)biphenyl, 2,2'-di-n-hexylbiphenyl, 4,4'-dicyclohexylbiphenyl, 4,4'-dibenzylbiphenyl, 4,4'-dicyanobiphenyl, 4,4'-bis(trifluoromethyl)biphenyl, 2,2'-bis(trifluoromethyl)biphenyl, 4,4'-bis(cyanomethyl)biphenyl, 3,3'-dimethoxybiphenyl, 4,4'-dimethoxybiphenyl, 2,2',3,3'-tetramethoxybiphenyl, 2,2',4,4'-tetramethoxybiphenyl, 2,2',5,5'-tetramethoxybiphenyl, 2,2'-diethoxybiphenyl, 3,3'-di-n-propoxybiphenyl, 4,4'-diisopropoxybiphenyl, 5,5'-di-n-butoxybiphenyl, 4,4'-di-tert-butoxybiphenyl, 4,4'-diphenoxybiphenyl, 4,4'-dibenzyloxybiphenyl, 4,4'-bis(methoxymethyl)biphenyl, 4,4'-bis(n-butoxymethyl)biphenyl, 4,4'-bis(methoxymethoxy)biphenyl, 4,4'-bis(benzyloxymethoxy)biphenyl, 4,4'-bis{2-(n-butoxy)ethoxy}biphenyl, 4,4'-diacetylbiphenyl, 4,4'-dibenzoylbiphenyl, 4,4'-bis(phenylsulfonyl)biphenyl, dimethyl biphenyl-4,4'-disulfonate, diethyl biphenyl-4,4'-disulfonate, di(2,2-dimethylpropyl) biphenyl-4,4'-disulfonate, di(2,2-dimethylpropyl) biphenyl-3,3'-disulfonate, 1,1'-binaphthalene, 2,2'-bithiophene, 3,3'-dihexyl-5,5'-bithiophene, 1,1'-dimethyl-5,5'-bi-pyrrole, 2,2'-bipyzidine, 3,3'-dimethyl-2,2'-bipyridine, 3,3'-dihexyl-5,5'-bipyridine, 2,2'-bipyrimidine, 5,5'-biquinoline, 1,1'-biisoquinoline, 4,4'-bis(2,1,3-benzothiadiazole) and 7,7'-bis(benzimidazole).

In a case where an aromatic compound (A) is reacted with an aromatic compound (A) having the same structure as that of the above-mentioned aromatic compound (A) and the aromatic compound (2) wherein n is 2 is used as the aromatic compound (A), the conjugated aromatic compound having a repeating unit represented by the following formula (21): 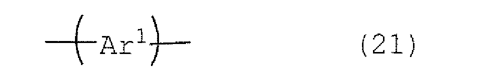 wherein Ar¹ is the same meaning as defined above, is obtained. Said conjugated aromatic compound usually contains 2 to 10,000 of the repeating unit represented by the formula (21), and the weight-average molecular weight thereof equivalent to
polystyrene is usually 500 to 3,000,000.

Specific examples of the repeating unit represented by the formula (21) include the repeating units represented by the following formulae (21a) to (21d).

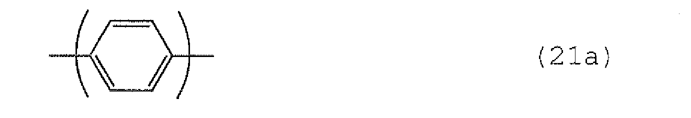

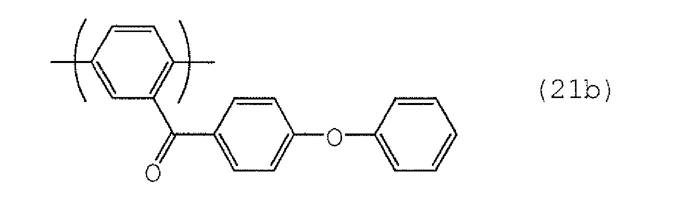

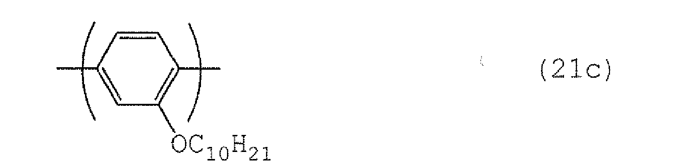

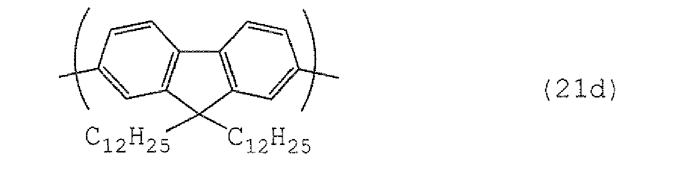

In a case where an aromatic compound (A) is reacted with an aromatic compound (A) having the same structure as that of the above-mentioned aromatic compound (A) and the aromatic compound (3) is used as the aromatic compound (A), the conjugated aromatic compound having a repeating unit represented by the following formula (22): 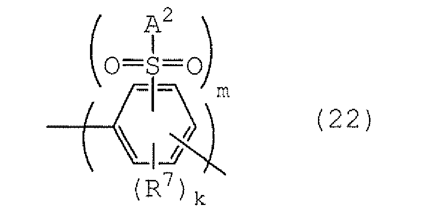 wherein A², R⁷, k and m are the same meanings as defined above, is obtained. Said conjugated aromatic compound usually contains 2 to 10,000 of the repeating unit represented by the formula (22), and the weight-average molecular weight thereof equivalent to polystyrene is usually 500 to 3,000,000.

Specific examples of the repeating unit represented by the formula (22) include the repeating units represented by the following formulae (22a) to (22e).

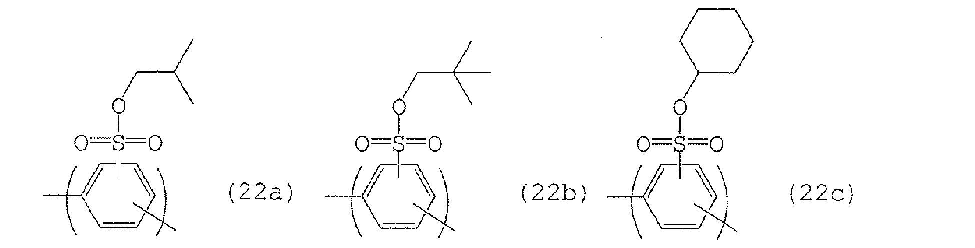

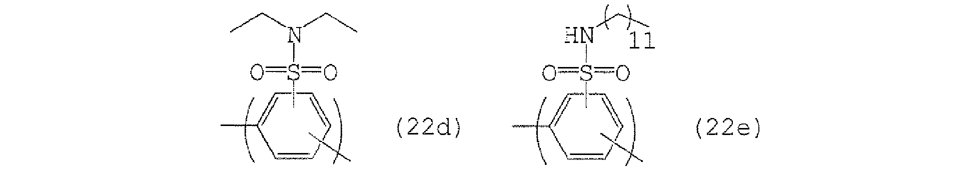

In a case where an aromatic compound (A) is reacted with an aromatic compound (A) having the same structure as that of the above-mentioned aromatic compound (A) and the aromatic compound (4) is used as the aromatic compound (A), the conjugated aromatic compound having a repeating unit represented by the following formula (23): 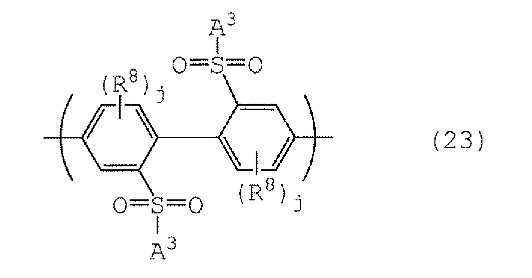 is obtained. Said conjugated aromatic compound usually contains 2 to 10,000 of the repeating unit represented by the formula (23), and the weight-average molecular weight thereof equivalent to polystyrene is usually 1,000 to 6,000,000.

Specific examples of the repeating unit represented by the formula (23) include the repeating units represented by the following formulae (23a) to (23e).

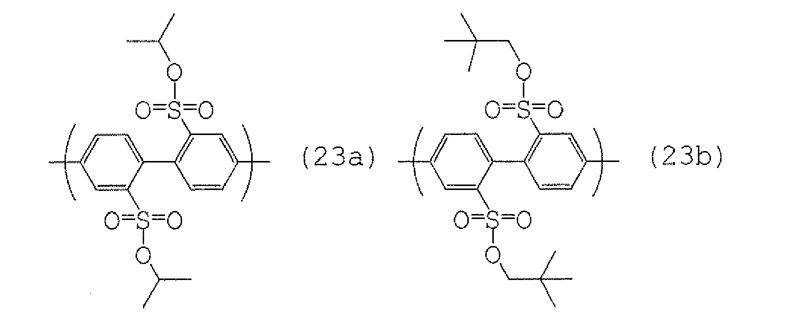

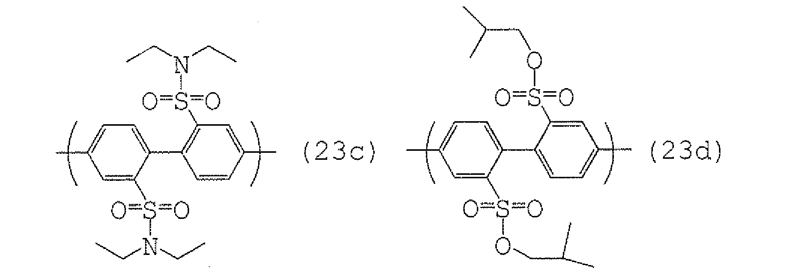

In a case where the aromatic compound (2) is used as the aromatic compound (A) and the aromatic compound (5) is used as the aromatic compound (B), a conjugated aromatic compound comprising the above-mentioned repeating unit represented by the formula (21) and a segment represented by the following formula (24): 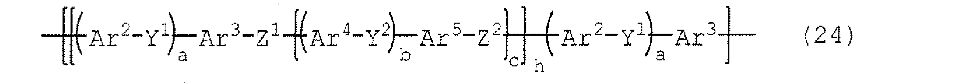 is obtained. The weight-average molecular weight thereof equivalent to polystyrene of said conjugated aromatic compound is usually 3,000 to 3,000,000.

Specific examples of the segment represented by the formula (24) include the following segments represented by the formulae (24a) to (24x). Additionally, in the following formulae, h represents the same meaning as defined above and is preferably an integer of 10 or more.

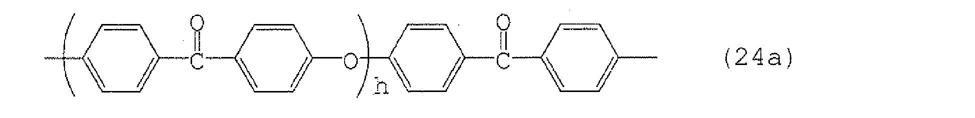

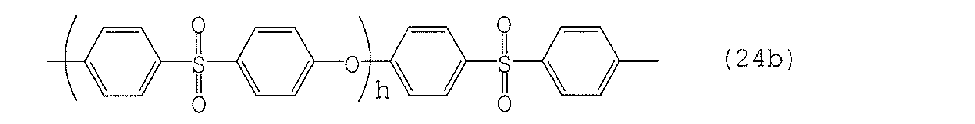

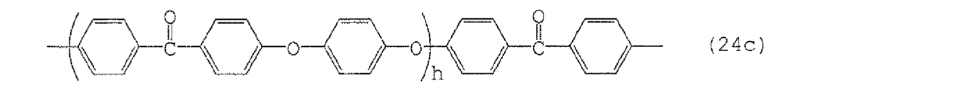

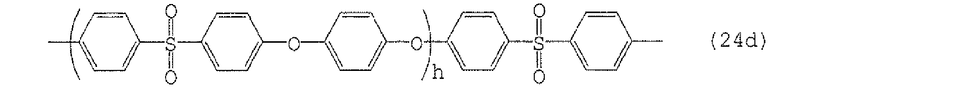

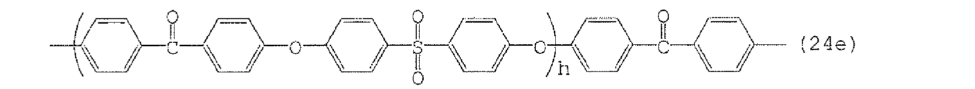

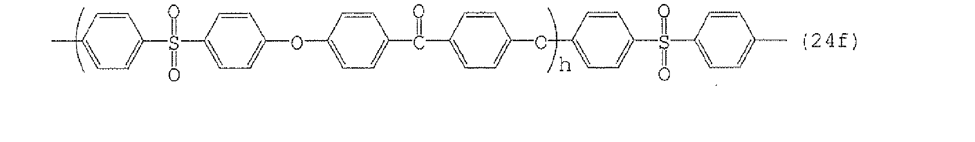

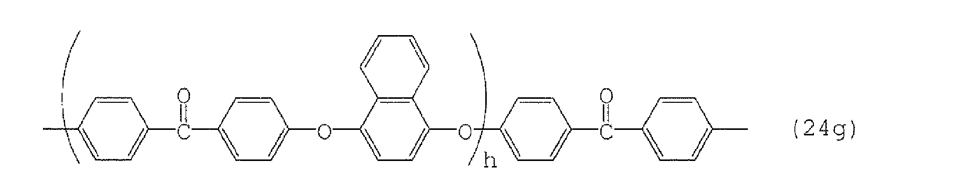

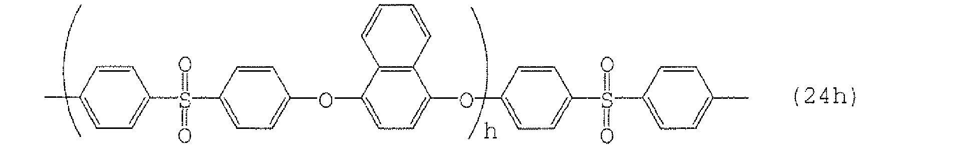

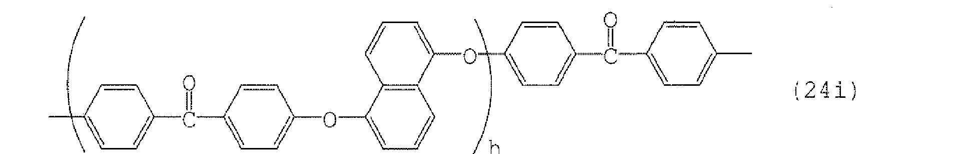

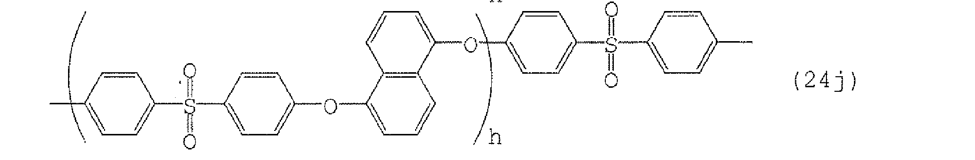

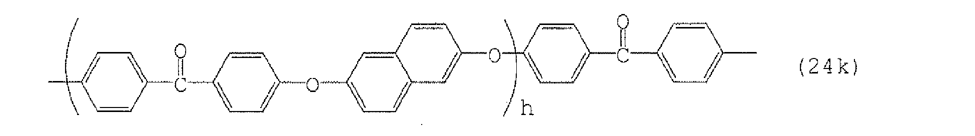

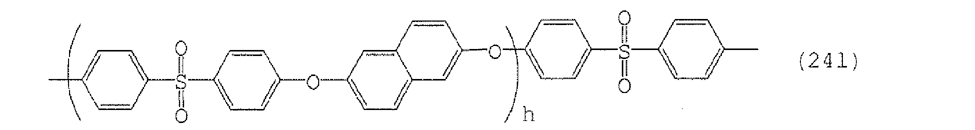

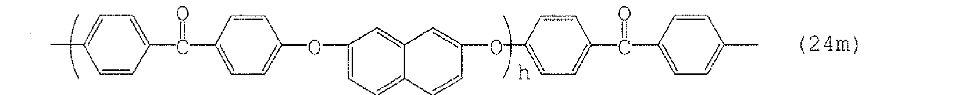

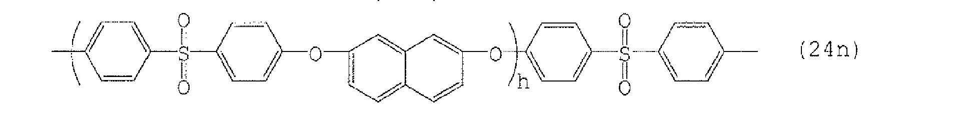

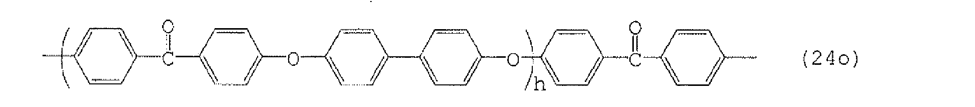

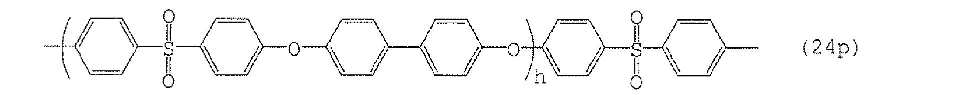

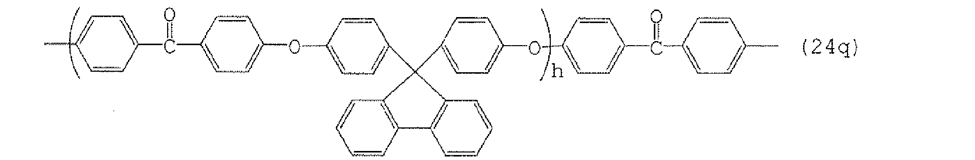

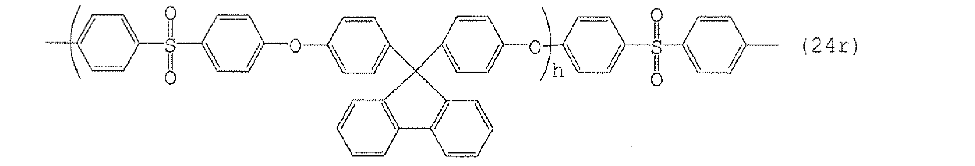

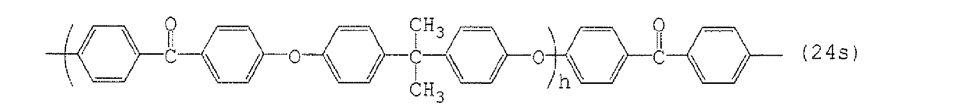

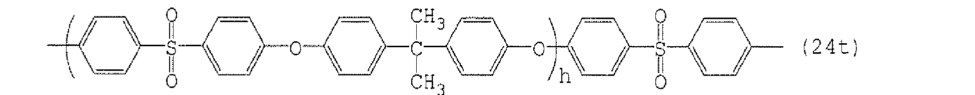

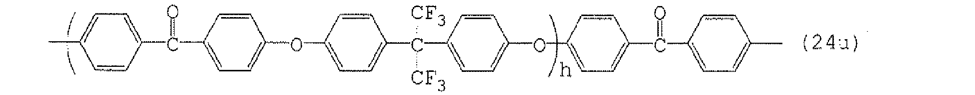

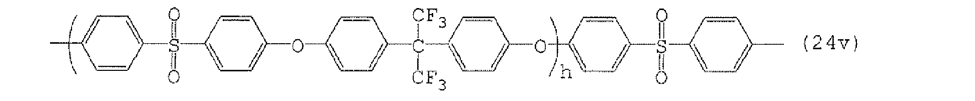

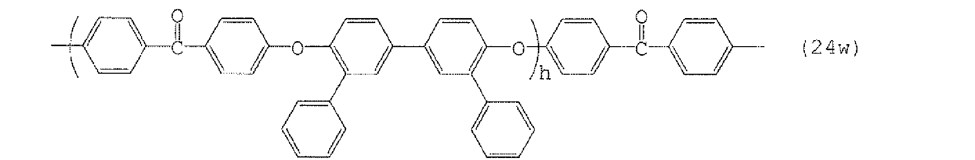

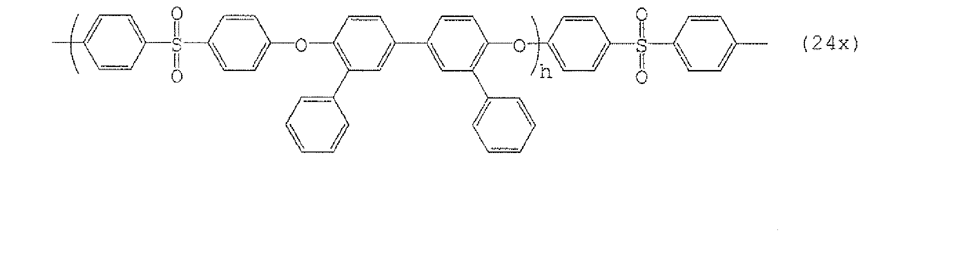

Examples of the conjugated aromatic compound comprising the repeating unit represented by the formula (21) and the segment represented by the formula (24) include a conjugated aromatic compound comprising any one repeating unit of the above-mentioned repeating units represented by the formulae (21a) to (21d) and any one segment of the above-mentioned segments represented by the formulae (24a) to (24x). Specific examples thereof include the following conjugated aromatic compounds represented by the formulae (I-1) to (1-16). Herein, in the following formulae, h represents the same meaning as defined above, and p represents an integer of 2 or more.

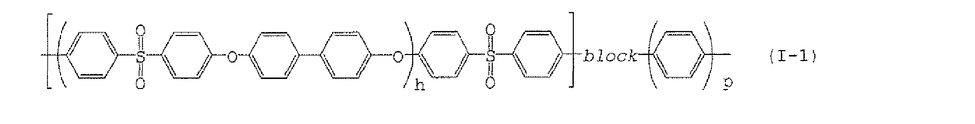

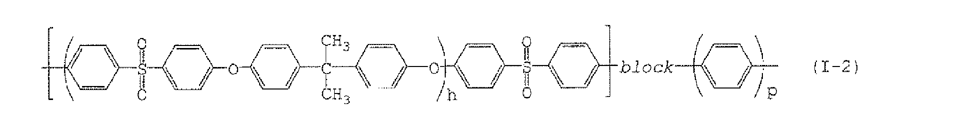

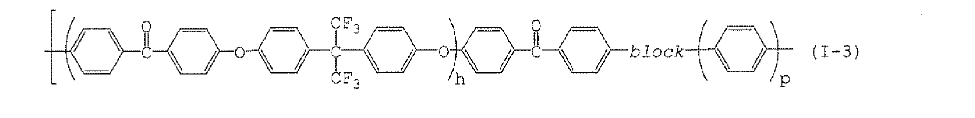

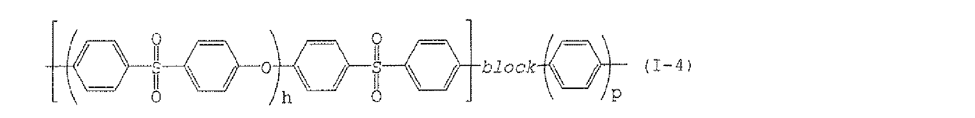

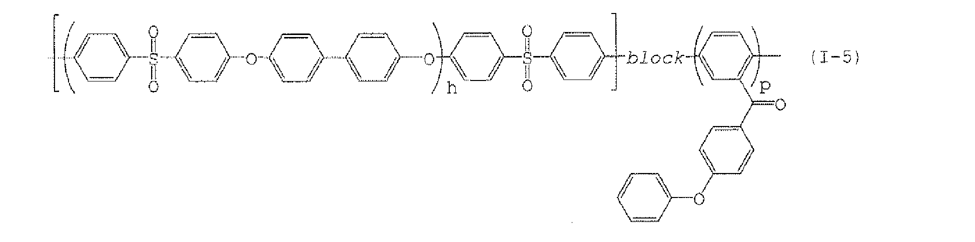

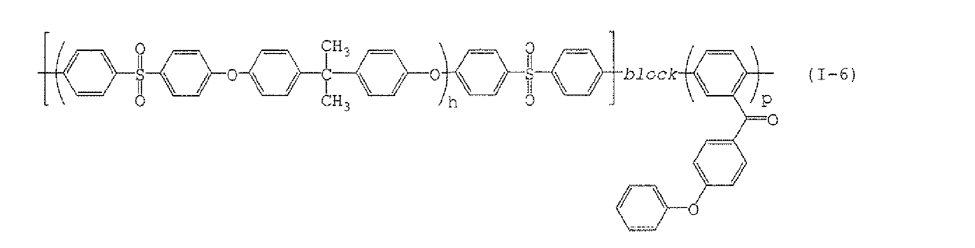

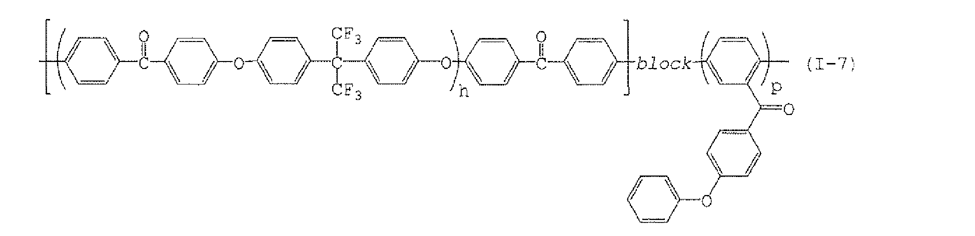

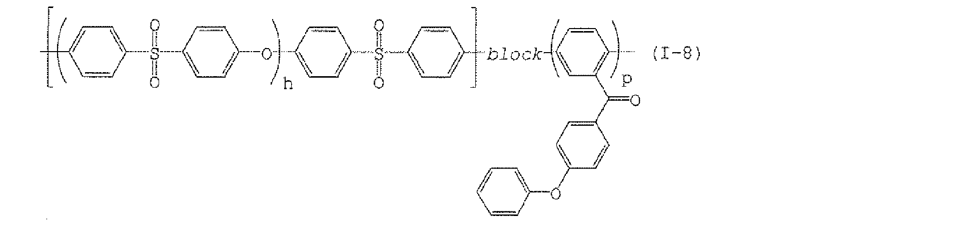

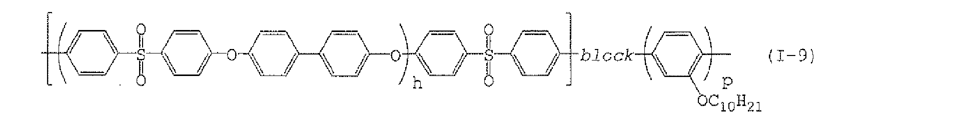

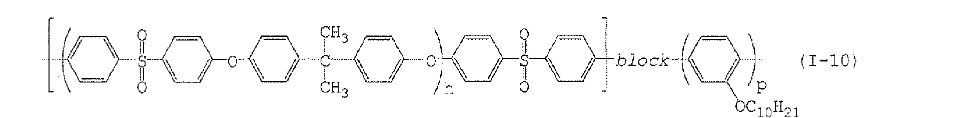

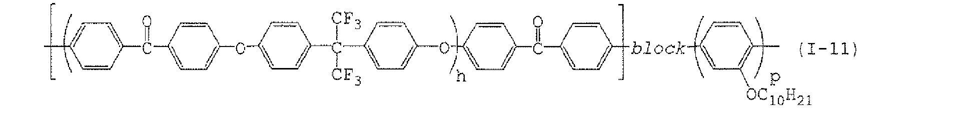

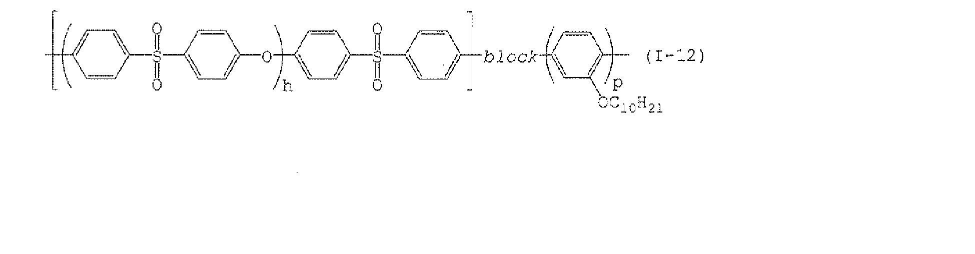

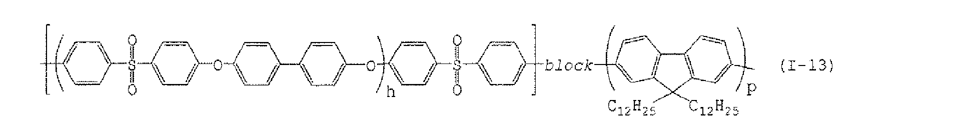

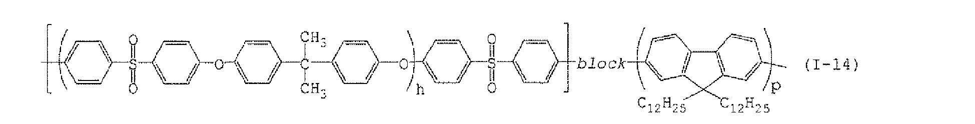

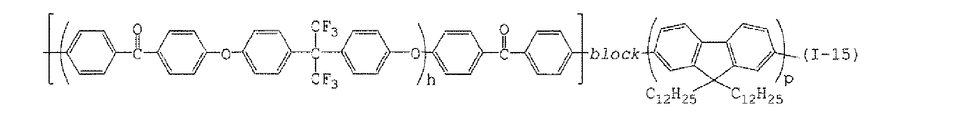

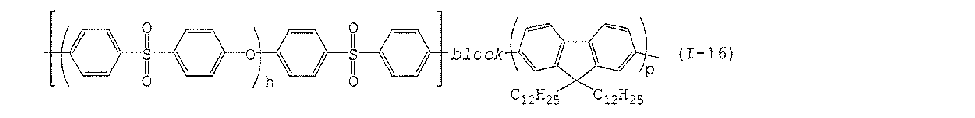

In a case where the aromatic compound (3) is used as the aromatic compound (A) and the aromatic compound (5) is used as the aromatic compound (B), a conjugated aromatic compound comprising the above-mentioned repeating unit represented by the formula (22) and a segment represented by the following formula (24) is obtained. The weight-average molecular weight thereof equivalent to polystyrene of the conjugated aromatic compound is usually 3,000 to 3,000,000. The amount of the repeating unit represented by the formula (22) in the conjugated aromatic compound is preferable 5% by weight or more and 95% by weight or less, and more preferably 30% by weight or more and 90% by weight or less, and the amount of the segment represented by the formula (24) is preferably 5% by weight or more and 95% by weight or less, and more preferably 10% by weight or more and 70% by weight or less.

Examples of the conjugated aromatic compound comprising the repeating unit represented by the formula (22) and the segment represented by the formula (24) include a conjugated aromatic compound comprising any one repeating unit of the above-mentioned repeating units represented by the formulae (22a) to (22e) and any one segment of the above-mentioned segments represented by the formulae (24a) to (24x). Specific examples thereof include the following conjugated aromatic compounds represented by the formulae (II-1) to (II-9). Herein, in the following formulae, h represents the same meanings as defined above and p represents an integer of 2 or more.

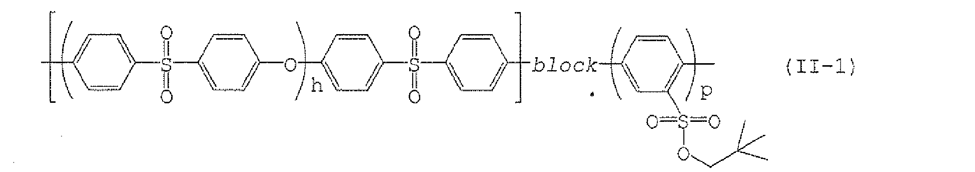

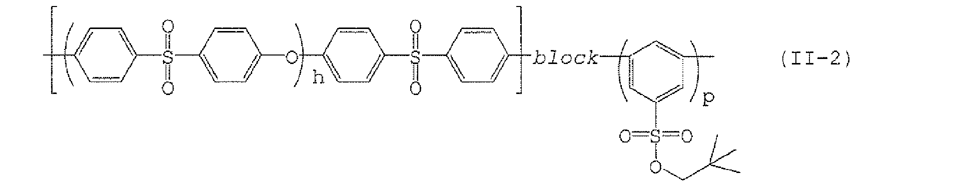

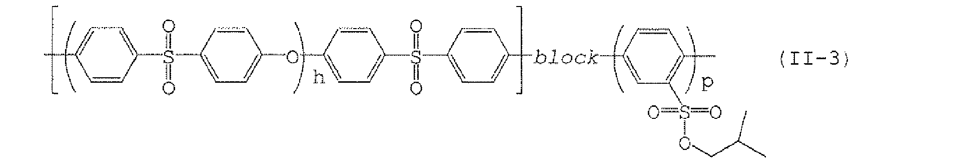

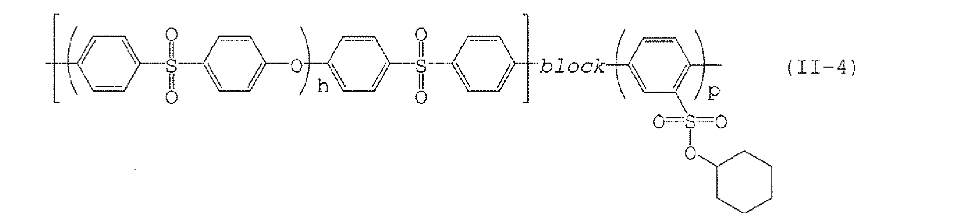

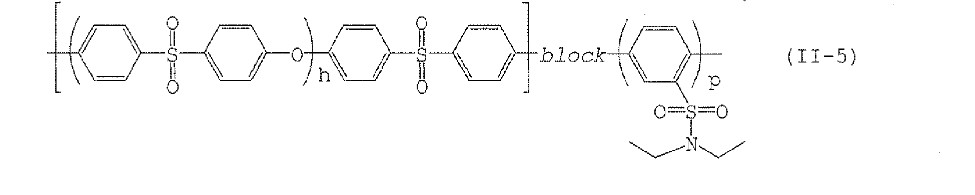

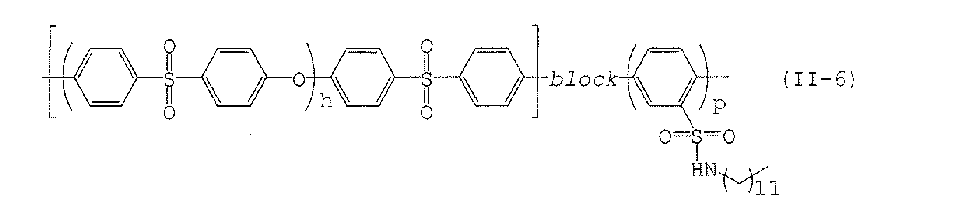

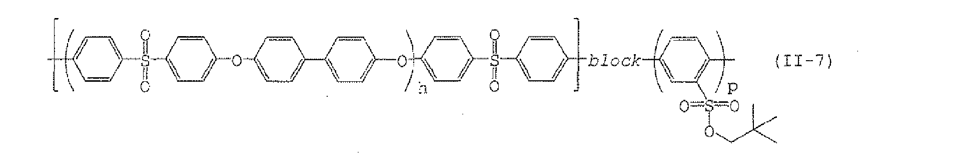

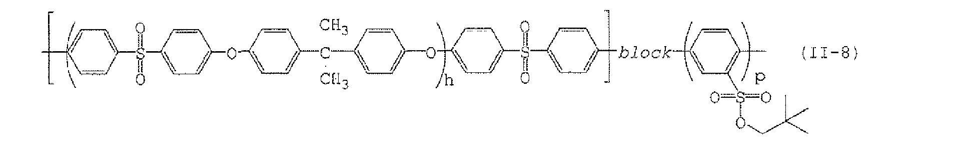

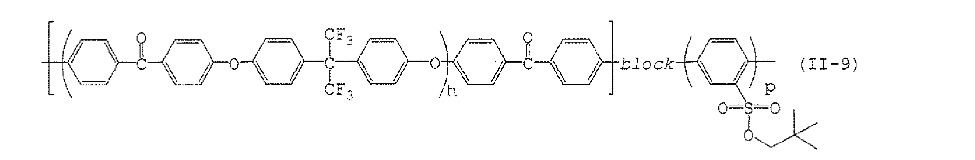

In a case where the aromatic compound (4) is used as the aromatic compound (A) and the aromatic compound (5) is used as the aromatic compound (B), a conjugated aromatic compound comprising the above-mentioned repeating unit represented by the formula (23) and a segment represented by the following formula (24) is obtained. The weight-average molecular weight thereof equivalent to polystyrene of the conjugated aromatic compound is usually 3,000 to 3,000,000. The amount of the repeating unit represented by the formula (23) in the conjugated aromatic compound is preferably 5% by weight or more and 95% by weight or less, and more preferably 30% by weight or more and 90% by weight or less, and the amount of the segment represented by the formula (24) is preferably 5% by weight or more and 95% by weight or less, and more preferably 10% by weight or more and 70% by weight or less.

Examples of the conjugated aromatic compound comprising the repeating unit represented by the formula (23) and the segment represented by the formula (24) include a conjugated aromatic compound comprising any one repeating unit of the above-mentioned repeating units represented by the formulae (23a) to (23d) and any one segment of the above-mentioned segments represented by the formulae (24a) to (24x). Specific examples thereof include the following conjugated aromatic compounds represented by the formulae (III-1) to (III-6). Herein, in the following formulae, h represents the same meanings as defined above and p represents an integer of 2 or more.

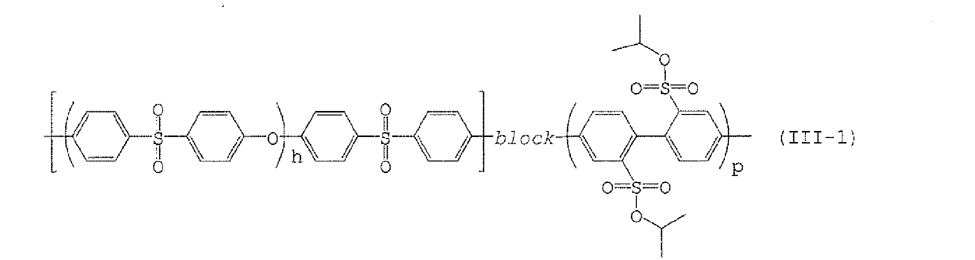

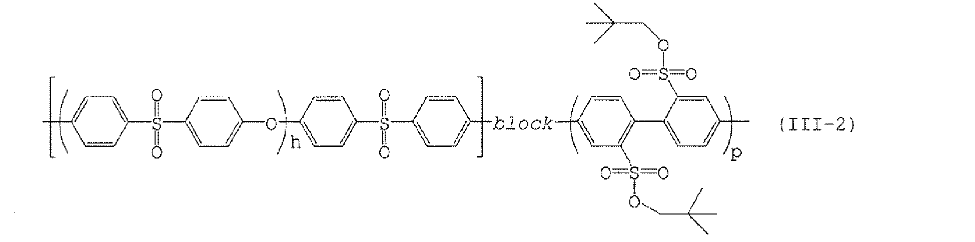

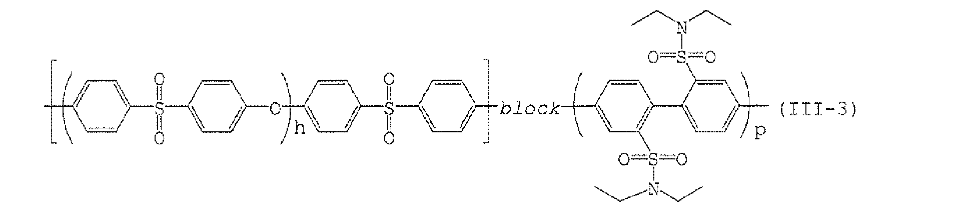

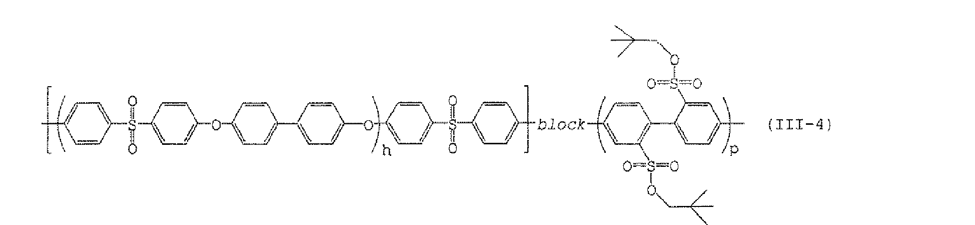

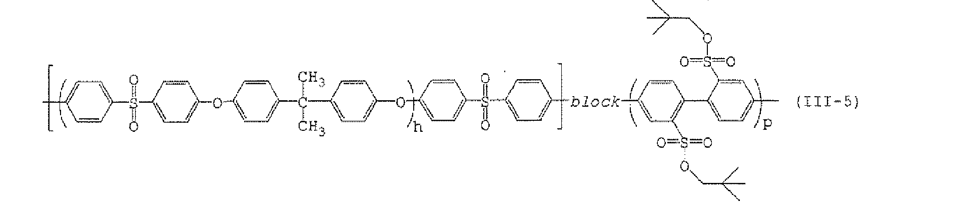

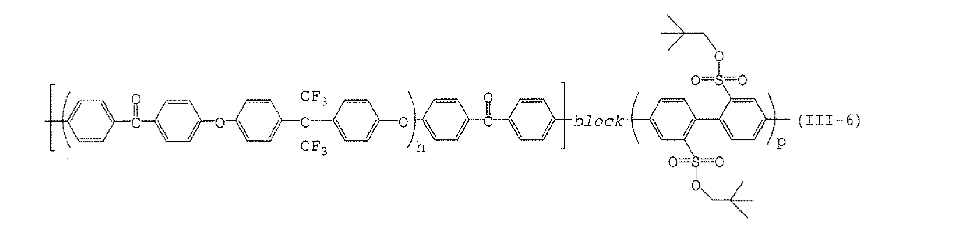

In a case where the aromatic compound (2) is used as the aromatic compound (A) and the aromatic compound (3) is used as the aromatic compound (B), a conjugated aromatic compound comprising the above-mentioned repeating unit represented by the formula (21) and the above-mentioned repeating unit represented by the following formula (22) is obtained. The weight-average molecular weight thereof equivalent to polystyrene of the conjugated aromatic compound is usually 1,000 to 2,000,000. The amount of the repeating unit represented by the formula (21) in the conjugated aromatic compound is preferably 1% by weight or more and 99% by weight or less, and the amount of the repeating unit represented by the formula (22) is preferably 1% by weight or more and 99% by weight or less.

Examples of the conjugated aromatic compound comprising the repeating unit represented by the formula (21) and the repeating unit represented by the formula (22) include a conjugated aromatic compound comprising any one repeating unit of the above-mentioned repeating units represented by the formulae (21a) to (21d) and any one segment of the above-mentioned repeating units represented by the formulae (22a) to (22e). Specific examples thereof include the following conjugated aromatic compounds represented by the formulae (IV-1) to (IV-4).

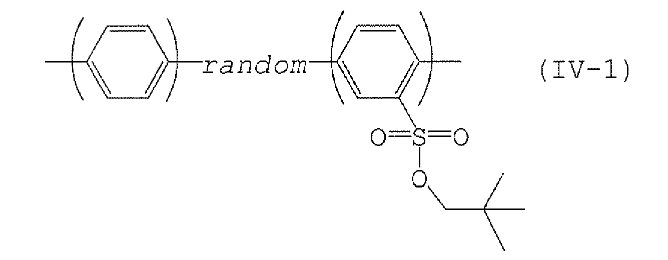

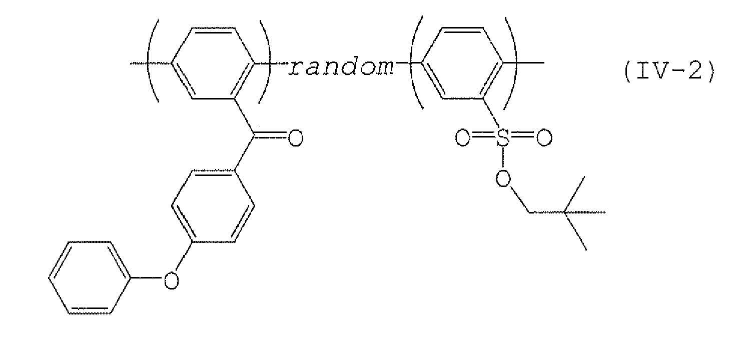

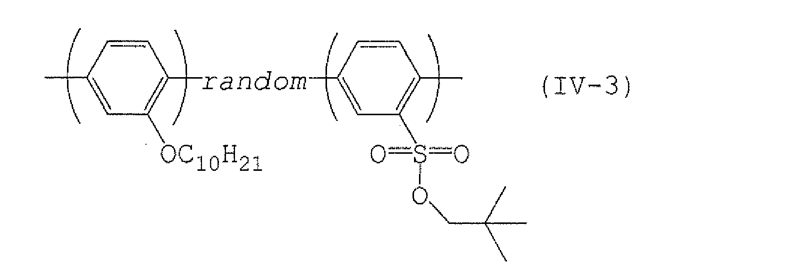

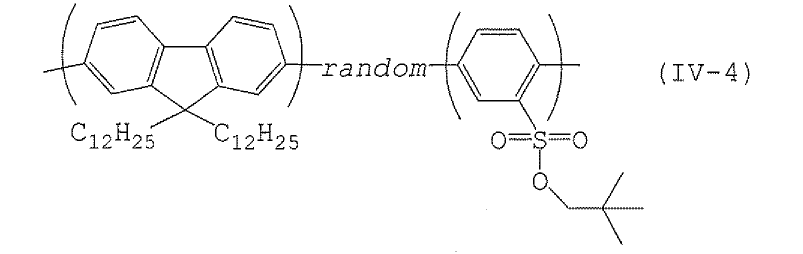

In a case where the aromatic compound (2) is used as the aromatic compound (A) and the aromatic compound (4) is used as the aromatic compound (B), a conjugated aromatic compound comprising the above-mentioned repeating unit represented by the formula (21) and the above-mentioned repeating unit represented by the following formula (23) is obtained. The weight-average molecular weight thereof equivalent to polystyrene of the conjugated aromatic compound is usually 1,000 to 2,000,000. The amount of the repeating unit represented by the formula (21) in the conjugated aromatic compound is preferably 1% by weight or more and 99% by weight or less, and the amount of the repeating unit represented by the formula (23) is preferably 1% by weight or more and 99% by weight or less.

Examples of the conjugated aromatic compound comprising the repeating unit represented by the formula (21) and the repeating unit represented by the formula (23) include a conjugated aromatic compound comprising any one repeating unit of the above-mentioned repeating units represented by the formulae (21a) to (21d) and any one segment of the above-mentioned repeating units represented by the formulae (23a) to (23d). Specific examples thereof include the following conjugated aromatic compounds represented by the formulae (V-1) to (V-4) .

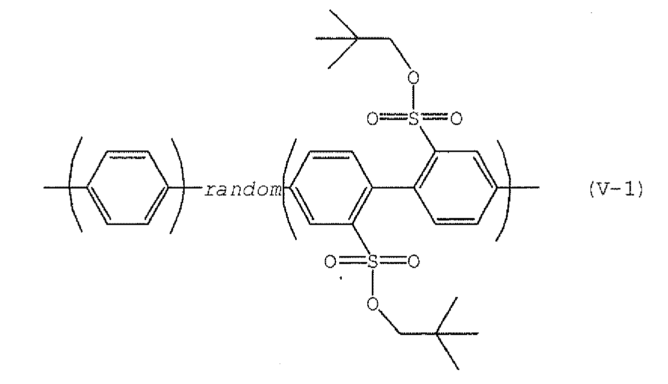

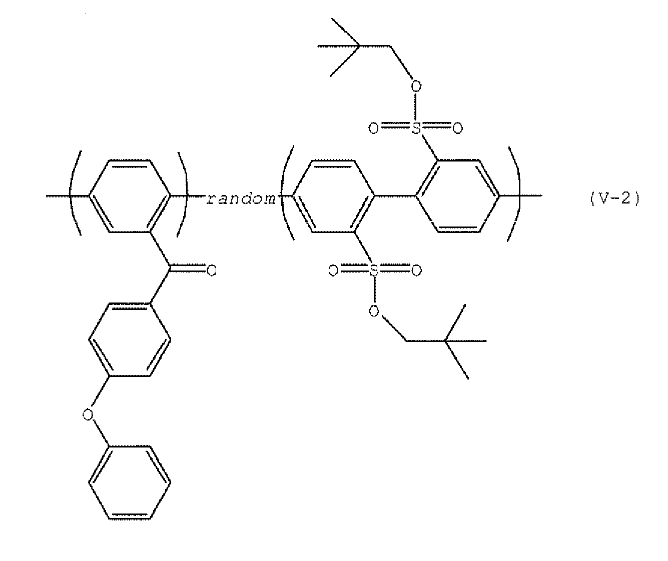

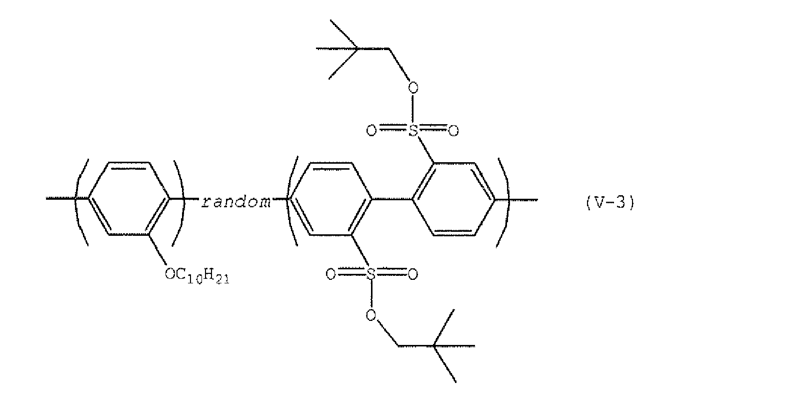

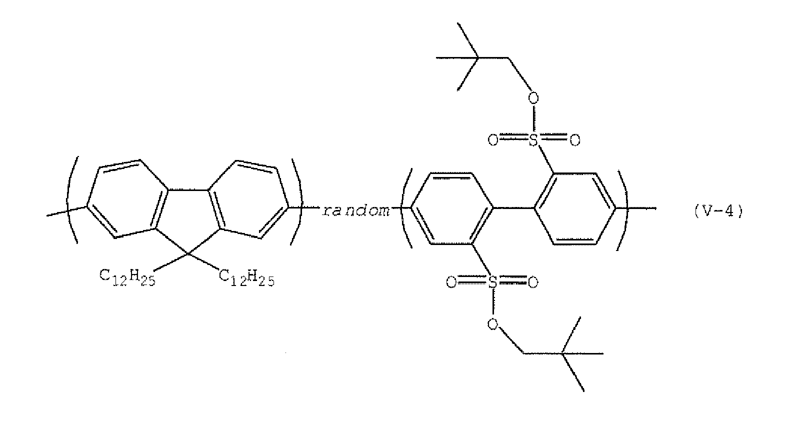

The content of each repeating unit in the conjugated aromatic compound comprising two or more kinds of the repeating unit can be adjusted by arbitrarily adjusting the used amount of the aromatic compounds used.

The conjugated aromatic compound comprising the repeating unit represented by the formula (22) or (23) can be used as a law material for synthesizing a polyelectrolyte for a polymer electrolyte fuel cell, and the preferable weight-average molecular weight equivalent to polystyrene in such case is 2,000 to 1,000,000 and more preferable one is 3,000 to 800,000.

### Examples

The present invention will be further illustrated by Examples in more detail below, but the present invention is not limited to these Examples. When the obtained conjugated aromatic compound was not a polymer, it was analyzed according to gas chromatography internal standard method and liquid chromatography internal standard method, and the yield thereof was calculated from their results. When the obtained conjugated aromatic compound was a polymer, it was analyzed with gel permeation chromatography (hereinafter, simply referred to as GPC), of which analytical condition was as followed, and the weight-average molecular weight (Mw) and the number-average molecular weight (Mn) thereof were calculated from its result.

### <Analytical Condition>

GPC measuring apparatus: CTO-10A (manufactured by Shimadzu Corporation)
Column: TSK-GEL (manufactured by Tosoh Coporation)
Column temperature: 40°C
Eluent: N,N-dimethylacetamide containing lithium bromide
(concentration of lithium bromide: 10 mmol/dm³)
Flow rate: 0.5 mL/minute
Detection wavelength: 300 nm

### [Example 1]

To a reaction container made of glass and equipped with a cooling apparatus, 15 mg of nickel bromide, 13 mg of 2,2'-bi-pyridine, 76 mg of zinc powder, 366 mg of di(2,2-dimethylpropyl) 4,4'-dichlorobiphenyl-2,2'-disulfonate, 18 mg of manganese (II) chloride and 5 mL of N,N-dimethylacetamide were added in an atmosphere of nitrogen at room temperature. The obtained mixture was stirred at 70°C for 4 hours to obtain a reaction mixture containing a conjugated aromatic compound consisting of a repeating unit represented by the following formula (i) 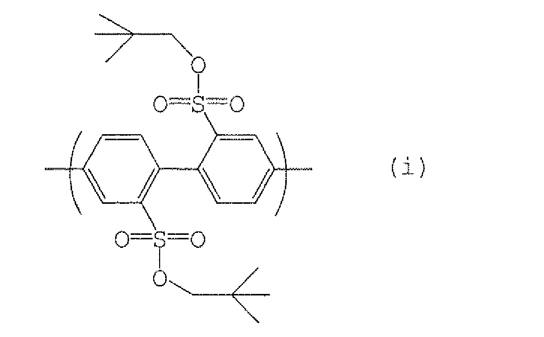 was obtained. Mw of the conjugated aromatic compound was 174,000, and Mn thereof was 52,000.

### [Example 2]

The reaction was conducted according to the same manner as that of Example 1, except that 30 mg of manganese(II) bromide was used in place of 18 mg of manganese (II) chloride, to obtain a reaction mixture containing a conjugated aromatic compound consisting of a repeating unit represented by the above-mentioned formula (i). Mw of the conjugated aromatic compound was 173,000, and Mn thereof was 53,000.

### [Comparative Example 1]

The reaction was conducted according to the same manner as that of Example 1, except that 18 mg of manganese(II) chloride was not used, to obtain a reaction mixture containing a conjugated aromatic compound consisting of a repeating unit represented by the above-mentioned formula (i). Mw of the conjugated aromatic compound was 67,000, and Mn thereof was 26,000.

### [Example 3]

To a reaction container made of glass and equipped with a cooling apparatus, 8 mg of nickel bromide, 18 mg of triphenylphosphine, 72 mg of zinc powder, 366 mg of di(2,2-dimethylpropyl) 4,4'-dichlorobiphenyl-2,2'-disulfonate, 15 mg of manganese (II) bromide and 5 mL of N,N-dimethylacetamide were added in an atmosphere of nitrogen at room temperature. The obtained mixture was stirred at 70°C for 4 hours to obtain a reaction mixture containing a conjugated aromatic compound consisting of a repeating unit represented by the above-mentioned formula (i) was obtained. Mw of the conjugated aromatic compound was 16,000, and Mn thereof was 10,000.

### [Comparative Example 2]

The reaction was conducted according to the same manner as that of Example 3, except that 15 mg of manganese(II) bromide was not used, to obtain a reaction mixture containing a conjugated aromatic compound consisting of a repeating unit represented by the above-mentioned formula (i).
Mw of the conjugated aromatic compound was 11,000, and Mn thereof was 8,000.

### [Example 4]

To a reaction container made of glass and equipped with a cooling apparatus, 8 mg of nickel bromide, 14 mg of 4,7-diphenyl-1,10-phenanthroline, 96 mg of zinc powder, 366 mg of di(2,2-dimethylpropyl) 4,4'-dichlorobiphenyl-2,2'-disulfonate, 13 mg of manganese(II) chloride and 5 mL of N,N-dimethylacetamide were added in an atmosphere of nitrogen at room temperature. The obtained mixture was stirred at 70°C for 4 hours to obtain a reaction mixture containing a conjugated aromatic compound consisting of a repeating unit represented by the above-mentioned formula (i) was obtained. Mw of the conjugated aromatic compound was 516,000, and Mn thereof was 147,000.

### [Comparative Example 3]

The reaction was conducted according to the same manner as that of Example 4, except that 13 mg of manganese (II) chloride was not used, to obtain a reaction mixture containing a conjugated aromatic compound consisting of a repeating unit represented by the above-mentioned formula (i).
Mw of the conjugated aromatic compound was 284,000, and Mn thereof was 89,000.

### [Example 5]

To a reaction container made of glass and equipped with a cooling apparatus, 8 mg of nickel bromide, 14 mg of 4,7-diphenyl-1,10-phenanthroline, 81 mg of manganese powder, 366 mg of di(2,2-dimethylpropyl) 4,4'-dichlorobiphenyl-2,2'-disulfonate, 13 mg of manganese(II) chloride and 5 mL of N,N-dimethylacetamide were added in an atmosphere of nitrogen at room temperature. The obtained mixture was stirred at 70°C for 4 hours to obtain a reaction mixture containing a conjugated aromatic compound consisting of a repeating unit represented by the above-mentioned formula (i) was obtained.
Mw of the conjugated aromatic compound was 443,000, and Mn thereof was 129,000.

### [Comparative Example 4]

The reaction was conducted according to the same manner as that of Example 5, except that 13 mg of manganese(II) chloride was not used, to obtain a reaction mixture containing a conjugated aromatic compound consisting of a repeating unit represented by the above-mentioned formula (i).

Mw of the conjugated aromatic compound was 364,000, and Mn thereof was 108,000.

### [Example 6]

To a reaction container made of glass and equipped with a cooling apparatus, 10 mg of nickel(0) bis(cyclooctadiene), 14 mg of 4,7-diphenyl-1,10-phenanthroline, 96 mg of zinc powder, 366 mg of di(2,2-dimethylpropyl)
4,4'-dichlorobiphenyl-2,2'-disulfonate, 13 mg of manganese(II) chloride and 5 mL of N,N-dimethylacetamide were added in an atmosphere of nitrogen at room temperature. The obtained mixture was stirred at 70°C for 4 hours to obtain a reaction mixture containing a conjugated aromatic compound consisting of a repeating unit represented by the above-mentioned formula (i) was obtained.
Mw of the conjugated aromatic compound was 231,000, and Mn thereof was 67,000.

### [Comparative Example 5]

The reaction was conducted according to the same manner as that of Example 6, except that 13 mg of manganese(II) chloride was not used, to obtain a reaction mixture containing a conjugated aromatic compound consisting of a repeating unit represented by the above-mentioned formula (i).
Mw of the conjugated aromatic compound was 138,000, and Mn thereof was 44,000.

### [Example 7]

To a reaction container made of glass and equipped with a cooling apparatus, 8 mg of nickel bromide, 19 mg of 1,1'-bis(diphenylphosphino) ferrocene, 72 mg of zinc powder, 366 mg of di(2,2-dimethylpropyl)
4,4'-dichlorobiphenyl-2,2'-disulfonate, 13 mg of manganese (II) chloride and 5 mL of N,N-dimethylacetamide were added in an atmosphere of nitrogen at room temperature. The obtained mixture was stirred at 70°C for 4 hours to obtain a reaction mixture containing a conjugated aromatic compound consisting of a repeating unit represented by the above-mentioned formula (i) was obtained.
Mw of the conjugated aromatic compound was 14,000, and Mn thereof was 10,000.

### [Comparative Example 6]

The reaction was conducted according to the same manner as that of Example 7, except that 13 mg of manganese(II) chloride was not used, to obtain a reaction mixture containing a conjugated aromatic compound consisting of a repeating unit represented by the above-mentioned formula (i).
Mw of the conjugated aromatic compound was 11,000, and Mn thereof was 8,000.

### [Example 8]

To a reaction container made of glass and equipped with a cooling apparatus, a solution obtained by dissolving 4.6 mg of nickel bromide, 8 mg of 4, 7-diphenyl-1, 10-phenanthroline, 70 mg of zinc powder, 366 mg of di(2,2-dimethylpropyl)
4,4'-dichlorobiphenyl-2,2'-disulfonate and 8 mg of manganese(II) chloride in 3 mL of ,N-dimethylacetamide and a solution obtained by dissolving 206 mg of SUMIKA EXCEL PES 5200P represented by the following formula: 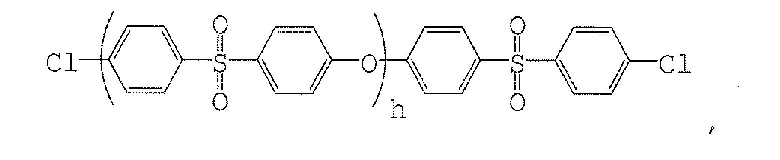 which had been manufactured by Sumitomo Chemical Company, Limited; Mw 94,000 and Mn 40,000 which had been measured by the above analytical condition, in 2 mL of N,N-dimethylacetamide were added in an atmosphere of nitrogen at room temperature. The obtained mixture was stirred at 70°C for 4 4 hours to obtain a reaction mixture containing a conjugated aromatic compound consisting of a repeating unit represented by the above-mentioned formula (i) and a segment represented by the following formula: 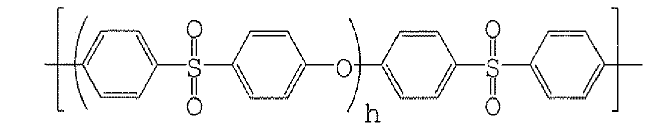 was obtained. Mw of the conjugated aromatic compound was 133,000, and Mn thereof was 48,000.

### [Comparative Example 7]

The reaction was conducted according to the same manner as that of Example 8, except that 8 mg of manganese(II) chloride was not used, to obtain a reaction mixture containing a conjugated aromatic compound consisting of a repeating unit represented by the above-mentioned formula (i) and the above-mentioned segment.
Mw of the conjugated aromatic compound was 81,000, and Mn thereof was 30,000.

### [Example 9]

To a reaction container made of glass and equipped with a cooling apparatus, 8 mg of nickel bromide, 7 mg of 2,2'-bipyridine, 9 mg of manganese chloride and 92 mg of zinc powder were added in an atmosphere of nitrogen at room temperature. To the obtained mixture, 89 mg of 4-chlorotoluene and 5 mL of N-methyl-2-pyrrolidone were added at room temperature. The obtained mixture was stirred at 70°C for 6 hours to obtain a reaction mixture containing 4,4'-dimethylbiphenyl was obtained. The yield of 4,4'-dimethylbiphenyl was 44 mg.

### [Comparative Example 8]

The reaction was conducted according to the same manner as that of Example 9, except that 9 mg of manganese(II) chloride was not used, to obtain a reaction mixture containing 4,4'-dimethylbiphenyl was obtained. The yield of 4,4'-dimethylbiphenyl was 31 mg.

### [Example 10]

To a reaction container made of glass and equipped with a cooling apparatus, 8 mg of nickel bromide, 8 mg of 1,10-phenanthroline, 9 mg of manganese chloride and 92 mg of zinc powder were added in an atmosphere of nitrogen at room temperature. To the obtained mixture, 89 mg of 4-chlorotoluene and 5 mL of N-methyl-2-pyrrolidone were added at room temperature. The obtained mixture was stirred at 70°C for 6 hours to obtain a reaction mixture containing 4,4'-dimethylbiphenyl was obtained. The yield of 4,4'-dimethylbiphenyl was 53 mg.

### [Comparative Example 9]

The reaction was conducted according to the same manner as that of Example 10, except that 9 mg of manganese(II) chloride was not used, to obtain a reaction mixture containing 4,4'-dimethylbiphenyl was obtained. The yield of 4,4'-dimethylbiphenyl was 44 mg.

### [Example 11]

To a reaction container made of glass and equipped with a cooling apparatus, 8 mg of nickel bromide, 18 mg of triphenylphosphine, 9 mg of manganese chloride and 92 mg of zinc powder were added in an atmosphere of nitrogen at room temperature. To the obtained mixture, 89 mg of 4-chlorotoluene and 2 mL of N-methyl-2-pyrrolidone were added at room temperature. The obtained mixture was stirred at 70°C for 6 hours to obtain a reaction mixture containing 4,4'-dimethylbiphenyl was obtained. The yield of 4,4'-dimethylbiphenyl was 27 mg.

### [Comparative Example 10]

The reaction was conducted according to the same manner as that of Example 11, except that 9 mg of manganese(II) chloride was not used, to obtain a reaction mixture containing 4,4'-dimethylbiphenyl was obtained. The yield of 4,4'-dimethylbiphenyl was 20 mg.

### [Example 12]

To a reaction container made of glass and equipped with a cooling apparatus, 5 mg of nickel bromide, 4 mg of 2,2'-bipyridine, 8 mg of manganese chloride and 92 mg of zinc powder were added in an atmosphere of nitrogen at room temperature. To the obtained mixture, 108 mg of 4'-chloroacetophenone and 5 mL of N-methyl-2-pyrrolidone were added at room temperature. The obtained mixture was stirred at 70°C for 6 hours to obtain a reaction mixture containing 4,4'-diacetylbiphenyl was obtained. The yield of 4,4'-diacetylbiphenyl was 71 mg.

### [Comparative Example 11]

The reaction was conducted according to the same manner as that of Example 12, except that 8 mg of manganese (II) chloride was not used, to obtain a reaction mixture containing 4,4'-diacetylbiphenyl was obtained. The yield of 4,4'-diacetylbiphenyl was 62 mg.

### [Example 13]

To a reaction container made of glass and equipped with a cooling apparatus, 8 mg of nickel bromide, 7 mg of 2,2'-bipyridine, 13 mg of manganese chloride and 92 mg of zinc powder were added in an atmosphere of nitrogen at room temperature. To the obtained mixture, 96 mg of 4-chlorobenzonitrile and 5 mL of N-methyl-2-pyrrolidone were added at room temperature. The obtained mixture was stirred at 70°C for 6 hours to obtain a reaction mixture containing 4,4'-dicyanobiphenyl was obtained. The yield of 4,4'-dicyanobiphenyl was 69 mg.

### [Comparative Example 12]

The reaction was conducted according to the same manner as that of Example 13, except that 13 mg of manganese(II) chloride was not used, to obtain a reaction mixture containing 4,4'-dcyanobiphenyl was obtained. The yield of 4,4'-dicyanobiphenyl was 39 mg.

### [Example 14]

To a reaction container made of glass and equipped with a cooling apparatus, 8 mg of nickel bromide, 7 mg of 2,2'-bipyridine, 13 mg of manganese chloride and 92 mg of zinc powder were added in an atmosphere of nitrogen at room temperature. To the obtained mixture, 91 mg of 4-chlorofluorobenzene and 5 mL of N-methyl-2-pyrrolidone were added at room temperature. The obtained mixture was stirred at 70°C for 6 hours to obtain a reaction mixture containing 4,4'-difluorobiphenyl was obtained. The yield of 4,4'-difluorobiphenyl was 53 mg.

### [Comparative Example 13]

The reaction was conducted according to the same manner as that of Example 14, except that 13 mg of manganese(II) chloride was not used, to obtain a reaction mixture containing 4,4'-difluorobiphenyl was obtained. The yield of 4,4'-difluorobiphenyl was 27 mg.

### [Example 15]

To a reaction container made of glass and equipped with a cooling apparatus, 8 mg of nickel bromide, 7 mg of 2,2'-bipyridine, 9 mg of manganese chloride and 92 mg of zinc powder were added in an atmosphere of nitrogen at room temperature. To the obtained mixture, 100 mg of 4-chloroanisole and 5 mL of N-methyl-2-pyrrolidone were added at room temperature. The obtained mixture was stirred at 70°C for 6 hours to obtain a reaction mixture containing 4,4'-dimethoxybiphenyl was obtained. The yield of 4,4'-dimethoxybiphenyl was 54 mg.

### [Comparative Example 14]

The reaction was conducted according to the same manner as that of Example 15, except that 9 mg of manganese(II) chloride was not used, to obtain a reaction mixture containing 4,4'-dimethoxybiphenyl was obtained. The yield of 4,4'-dimethoxybiphenyl was 49 mg.

### [Example 16]

To a reaction container made of glass and equipped with a cooing apparatus, 8 mg of nickel bromide, 18 mg of triphenylphosphine, 9 mg of manganese chloride and 92 mg of zinc powder were added in an atmosphere of nitrogen at room temperature. To the obtained mixture, 100 mg of 3-chloroanisole and 2 mL of N-methyl-2-pyrrolidone were added at room temperature. The obtained mixture was stirred at 70°C for 6 hours to obtain a reaction mixture containing 3,3'-dimethoxybiphenyl was obtained. The yield of 3,3'-dimethoxybiphenyl was 59 mg.

### [Comparative Example 15]

The reaction was conducted according to the same manner as that of Example 16, except that 9 mg of manganese (II) chloride was not used, to obtain a reaction mixture containing 3,3'-dimethoxybiphenyl was obtained. The yield of 3,3'-dimethoxybiphenyl was 32 mg.

### [Example 17]

To a reaction container made of glass and equipped with a cooling apparatus, 8 mg of nickel bromide, 7 mg of 2,2'-bipyridine, 13 mg of manganese chloride and 92 mg of zinc powder were added in an atmosphere of nitrogen at room temperature. To the obtained mixture, 114 mg of 2-bromothiophene and 5 mL of N-methyl-2-pyrrolidone were added at room temperature. The obtained mixture was stirred at 70°C for 6 hours to obtain a reaction mixture containing 2,2'-bithiophene was obtained. The yield of 2,2'-bithiopl-2ene was 15 mg.

### [Comparative Example 16]

The reaction was conducted according to the same manner as that of Example 27, except that 13 mg of manganese(II) chloride was not used, to obtain a reaction mixture containing 2,2'-bithiophene was obtained. The yield of 2,2'-bithiophene was 10 mg.

### [Example 18]

To a reaction container made of glass and equipped with a cooling apparatus, 8 mg of nickel bromide, 7 mg of 2,2'-bipyridine, 13 mg of manganese chloride and 92 mg of zinc powder were added in an atmosphere of nitrogen at room temperature. To the obtained mixture, 115 mg of 2-chloroquinoline and 5 mL of N-methyl-2-pyrrolidone were added at room temperature. The obtained mixture was stirred at 70°C for 6 hours to obtain a reaction mixture containing 2,2'-biquinoline was obtained. The yield of 2,2'-biquinoline was 85 mg.

### [Comparative Example 17]

The reaction was conducted according to the same manner as that of Example 18, except that 13 mg of manganese(II) chloride was not used, to obtain a reaction mixture containing 2,2'-biquinoline was obtained. The yield of 2,2'-biquinoline was 69 mg.

### Industrial Applicability

According to the present invention, a conjugated aromatic compound can be produced more advantageously.

## Claims

1. A method for producing a conjugated aromatic compound comprising reacting an aromatic compound (A) wherein one or two leaving groups are bonded to an aromatic ring with an aromatic compound (A) having the same structure as that of the above-mentioned aromatic compound (A) or an aromatic compound (B) being structurally different from the above-mentioned aromatic compound (A) and having one or two leaving groups bonded to an aromatic ring, in the presence of a nickel compound, a ligand, a manganese salt and a metal reducing agent.

2. The method according to claim 1, wherein the aromatic rings of the aromatic compounds (A) and (B) are independently a benzene ring, a biphenyl ring, a naphthalene ring, a fluorene ring, an anthracene ring, a phenanthrene ring, a thiophene ring, a pyrrole ring, a pyridine ring, a pyrimidine ring, a quinoline ring, an isoquinoline ring or a quinoxaline ring, and the aromatic ring may be substituted with at least one group uninvolved in the reaction.

3. The method according to claim 1, wherein an aromatic compound (A) is reacted with an aromatic compound (A) having the same structure as that of the aromatic compound (A).

4. The method according to claim 1, wherein the aromatic compound (A) is reacted with an aromatic compound (B) being structurally different from the aromatic compound (A).

5. The method according to claim 3 or 4, wherein the aromatic compound (A) is an aromatic compound represented by the formula (2):
Ar^{1_}(X¹)ₙ (2)
wherein Ar¹ represents an n-valent aromatic group, and the aromatic ring of which the above-mentioned aromatic group is composed is a benzene ring, a biphenyl ring, a naphthalene ring, a fluorene ring, an anthracene ring, a phenanthrene ring, a thiophene ring, a pyrrole ring, a pyridine ring, a pyrimidine ring, a quinoline ring, an isoquinoline ring or a quinoxaline ring, and may be substituted with at least one group uninvolved in the reaction, X¹ is independently in each occurrence a leaving group, and n represents 1 or 2.

6. The method according to claim 3 or 4, wherein the aromatic compound (A) is an aromatic compound represented by the formula (3): 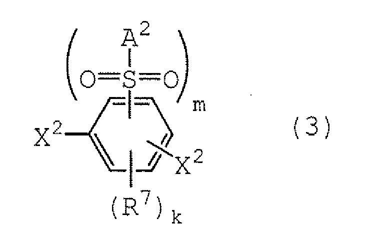 wherein A² represents an amino group substituted with one or two C1-C20 hydrocarbon groups, or a C1-C20 alkoxy group, and the above-mentioned hydrocarbon and alkoxy groups may be substituted with at least one group selected from the group consisting of a fluorine atom, a C1-C20 alkoxy group, a C6-C20 aryl group, a C6-C20 aryloxy group, a C2-C20 acyl group and a cyano group, R⁷ is independently in each occurrence a hydrogen atom, a fluorine atom, a C1-C20 alkyl group, a C1-C20 alkoxy group, a C6-C20 aryl group, a C6-C20 aryloxy group, a C2-C20 acyl group or a cyano group, and the above-mentioned C1-C20 alkyl, C1-C20 alkoxy, C6-C20 aryl, C6-C20 aryloxy and C2-C20 acyl groups may be substituted with at least one substituent selected from the group consisting of a fluorine atom, a cyano group, a C1-C20 alkoxy group, a C6-C20 aryl group and a C6-C20 aryloxy group, and the neighboring two R⁷s may be bonded to form a ring, X² represents a chlorine atom, a bromine atom or an iodine atom,
and m represents 1 or 2 and k represents 4-m.

7. The method according to claim 3 or 4, wherein the aromatic compound (A) is an aromatic compound represented by the formula (4): 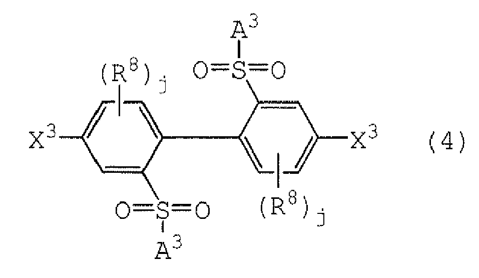 wherein A³ represents an amino group substituted with one or two C1-C20 hydrocarbon groups, or a C3-C20 alkoxy group, and the above-mentioned hydrocarbon and alkoxy groups may be substituted with at least one group selected from the group consisting of a fluorine atom, a C1-C20 alkoxy group, a C6-C20 aryl group, a C6-C20 aryloxy group, a C2-C20 acyl group and a cyano group, R⁸ is independently in each occurrence a fluorine atom, a C1-C20 alkyl group, a C1-C20 alkoxy group, a C6-C20 aryl group, a C6-C20 aryloxy group, a C2-C20 acyl group or a cyano group, and the above-mentioned C1-C20 alkyl, C1-C20 alkoxy, C6-C20 aryl, C6-C20 aryloxy and C2-C20 acyl groups may be substituted with at least one substituent selected from the group consisting of a fluorine atom, a cyano group, a C1-C20 alkoxy group, a C6-C20 aryl group and a C6-C20 aryloxy group, and the neighboring two R⁸s may be bonded to form a ring,
X³ represents a chlorine atom, a bromine atom or an iodine atom, and j represents an integer of 0 to 3.

8. The method according to claim 4, wherein an aromatic compound represented by the formula (2):
Ar^{1_}(X¹)ₙ (2)
wherein Ar¹, X¹ and n are the same as defined in claim 5, is used as the aromatic compound (A), and an aromatic compound represented by the formula (2), an aromatic compound represented by the formula (3): 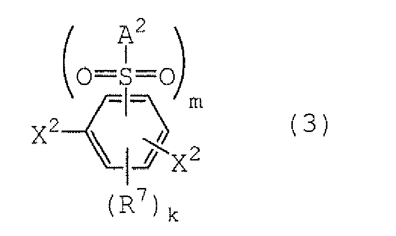 wherein A², R⁷, X², m and k are the same as defined in claim 6, an aromatic compound represented by the formula (4): 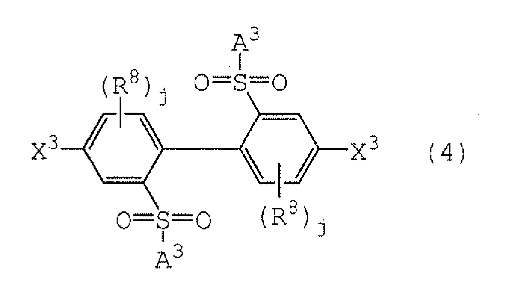 wherein A³, R⁸, X³ and j are the same as defined in claim 7, or an aromatic compound represented by the formula (5): 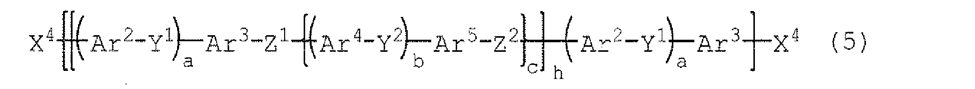 wherein a, b and c are the same or different and represent 0 or 1, and h represents an integer of 5 or more,
Ar², Ar³, Ar⁴ and Ar⁵ each independently represent a divalent aromatic group, and the divalent aromatic group may be substituted with at least one substituent selected from the group consisting of the following (a2) to (e2):
(a2) a C1-C20 alkyl group which may be substituted with at least one substituent selected from the group consisting of a fluorine atom, a cyano group, a C1-C20 alkoxy group, a C6-C20 aryl group and a C6-C20 aryloxy group;
(b2) a C1-C20 alkoxy group which may be substituted with at least one substituent selected from the group consisting of a fluorine atom, a cyano group, a C1-C20 alkoxy group, a C6-C20 aryl group and a C6-C20 aryloxy group;
(c2) a C6-C20 aryl group which may be substituted with at least one substituent selected from the group consisting of a fluorine atom, a cyano group, a C1-C20 alkoxy group and a C6-C10 aryloxy group;
(d2) a C6-C20 aryloxy group which may be substituted with at least one substituent selected from the group consisting of a fluorine atom, a cyano group, a C1-C20 alkoxy group and a C6-C20 aryloxy group; and
(e2) a C2-C20 acyl group which may be substituted with at least one substituent selected from the group consisting of a fluorine atom, a cyano group, a C1-C20 alkoxy group, a C6-C20 aryl group and a C6-C20 aryloxy group, Y¹ and Y² each independently represent a single bond, -CO-, -SO₂--C(CH₃)₂-, -C(CF₃)₂- or a fluorene-9,9-diyl group,
Z¹ and Z² each independently represent -Q- or -S-, and X⁴ represents a chlorine atom, a bromine atom or an iodine atom, which are structurally different from the aromatic compound (A), is used as the aromatic compound (B).

9. The method according to claim 4, wherein an aromatic compound represented by the formula (3): 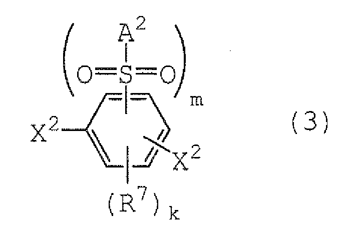 wherein A², R⁷, X², m and k are the same as defined in claim 6,
is used as the aromatic compound (A), and an aromatic compound represented by the formula (2): 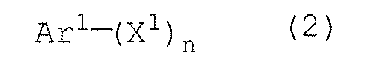 wherein Ar¹, X¹ and n are the same as defined in claim 5, an aromatic compound represented by the formula (3), an aromatic compound represented by the formula (4): 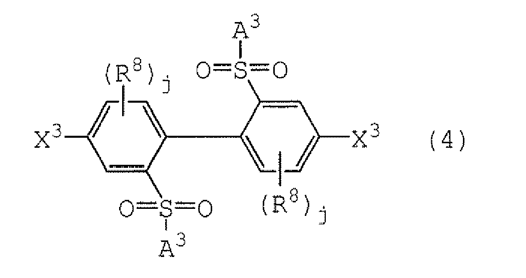 wherein A³, R⁸, X³ and j are the same as defined in claim 7, or an aromatic compound represented by the formula (5): 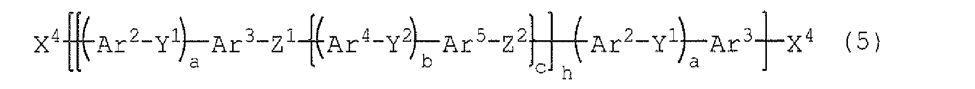 wherein a, b, c, h, Ar², Ar³, Ar⁴, Ar³, Y¹, Y², Z¹, Z² and X⁴ are the same as defined in claim 8, which are structurally different from the aromatic compound (A), is used as the aromatic compound (B) .

10. The method according to claim 4, wherein an aromatic compound represented by the formula (4): 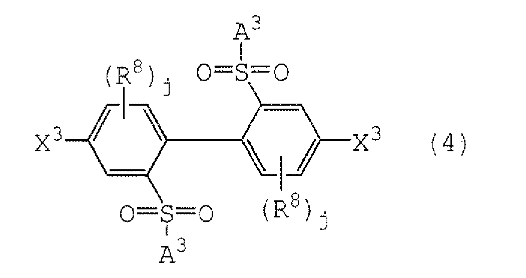 wherein A³, R⁸, X³ and j are the same as defined in claim 7, is used as the aromatic compound (A), and an aromatic compound represented by the formula (2):
Ar^{1_}(X¹)ₙ (2)
wherein Ar¹, X¹ and n are the same as defined in claim 5, an aromatic compound represented by the formula (3): 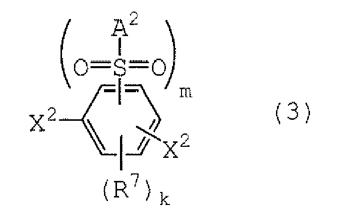 wherein A², R⁷, X², m and k are the same as defined in claim 6, an aromatic compound represented by the formula (4) or an aromatic compound represented by the formula (5): 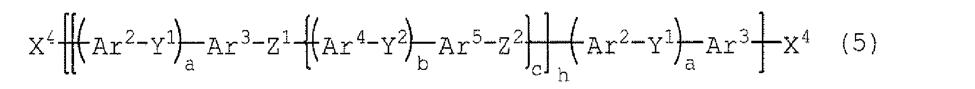 wherein a, b, c, h, Ar², Ar³, Ar⁴, Ar⁵, Y¹, Y², Z¹, Z² and X⁴ are the same as defined in claim 8, which are structurally different from the aromatic compound (A), is used as the aromatic compound (B).

11. The method according to claim 1, wherein the leaving group is a chlorine atom, a bromine atom or an iodine atom.

12. The method according to claim 1, wherein the nickel compound is a nickel halide.

13. The method according to claim 1, wherein the nickel compound is bis(cyclooctadiene)nickel(0).

14. The method according to claim 1, wherein the ligand is a ligand having a nitrogen atom or a phosphorus atom.

15. The method according to claim 1, wherein the manganese salt is a manganese(II) halide.

16. The method according to claim 1, wherein the metal reducing agent is zinc or manganese.
